# EUROPEAN PATENT SPECIFICATION

(11) **EP 4 345 103 B1**
(45) Date of publication and mention of the grant of the patent: **15.07.2026**
(21) Application number: 22832069.3
(22) Date of filing: 28.06.2022
(51) Int. Cl.: C07D 513/20, A61K 31/429, A61K 31/4015, A61P 35/00

(54) **THIAZOLE-LACTAM-SPIROHETEROCYCLIC COMPOUNDS AND APPLICATIONS THEREOF**
THIAZOL-LACTAM-SPIROHETEROCYCLISCHE VERBINDUNGEN UND ANWENDUNGEN DAVON
COMPOSÉS THIAZOLE-LACTAME-SPIROHÉTÉROCYCLIQUES ET LEURS APPLICATIONS

(30) Priority: 28.06.2021 CN 202110722003; 31.12.2021 CN 202111673614; 17.06.2022 CN 202210693548
(43) Date of publication of application: 03.04.2024
(73) Proprietor: D3 Bio (Wuxi) Co., Ltd., Wuxi, JiangSu 214092 (CN)
(72) Inventor: LI, Yi, Shanghai 200131 (CN); YU, Tao, Shanghai 200131 (CN); LIU, Ning, Shanghai 200131 (CN); WU, Chengde, Shanghai 200131 (CN); CHEN, Shuhui, Shanghai 200131 (CN)
(74) Representative: Cabinet Becker et Associés
(86) International application number: PCT/CN2022/102031
(87) International publication number: WO 2023/274256

(56) References cited:
- WO-A1-2019/223632
- WO-A1-2020/228817
- WO-A1-2021/110168
- WO-A1-2021/110169
- CN-A- 107 074 874
- CN-A- 107 108 648
- CN-A- 110 950 876

## Description

**This application claims the priority of:**
CN202110722003.6, filed on June 28, 2021;
CN202111673614.2, filed on December 31, 2021;
CN202210693548.3, filed on June 17, 2022.

### FIELD OF THE INVENTION

The present disclosure relates to a class of thiazololactamospiroheterocyclic compounds and use thereof in the manufacture of a medicament for treating related diseases. Specifically, the present disclosure relates to a compound represented by formula (I) and a pharmaceutically acceptable salt thereof.

### BACKGROUND OF THE INVENTION

Ras/Raf/MEK/ERK pathway is a classical mitogen activated protein kinase (MAPK) signaling cascade pathway, is involved in the signaling of various growth factors, cytokines, mitogens and hormone receptors after activation, and is one of the most important signaling pathways for controlling cell growth, differentiation and survival.

Studies have shown that abnormal activation of Ras/Raf/MEK/ERK pathway caused by mutation or amplification is a determinant of various cancers. In human tumors, the incidence of RAS mutation is about 22%, the incidence of BRAF mutation is about 7%, and the incidence of MEK mutation is about 1%. Therefore, key node proteins on this pathway have become important targets for the treatment of cancers (Cancer Discov. 2019, 9, 329-341). Currently, a number of BRAF inhibitors and MEK1/2 inhibitors, as well as their combination regimens, have been approved by the US FDA for the treatment of melanoma, BRAFV600E mutant non-small cell lung cancer and other cancers. However, the use of BRAF and MEK inhibitors for these upstream nodes can rapidly lead to a problem of drug resistance due to mutation or pathway reactivation, greatly limiting their clinical application.

Extracellular regulated protein kinases (ERK) (especially ERK1 and ERK2 kinases) are major players and downstream key nodes in the Ras/Raf/MEK/ERK pathway, and their over-activation can be found in many human cancers. ERK, as the terminal signaling kinase of this pathway, has not yet been found to have mutations that lead to drug resistance. Therefore, a drug targeting ERK kinase is expected to overcome the problem of drug resistance caused by the treatment with upstream target inhibitors, and become a more potential therapeutic strategy. But so far, research on ERK inhibitors is still in the clinical phase, and no ERK inhibitors have been approved for marketing as drugs. Known ERK inhibitors are for instance disclosed in: WO2021/110168 A1, WO2021/110169 A1, WO2019223632 A1, WO 2020/228817 A1.

In summary, there is an urgent need to develop a safe and effective ERK inhibitor drug to meet the need of treatment of a tumor.

### SUMMARY OF THE INVENTION

The present disclosure provides a compound represented by formula (I) or a pharmaceutically acceptable salt thereof, 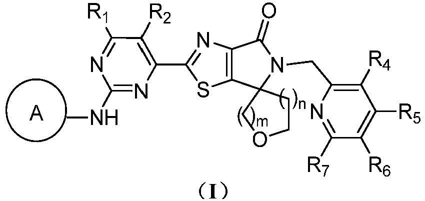 wherein
R₁ and R₂ are each independently selected from H and C₁₋₃ alkyl, wherein the C₁₋₃ alkyl is optionally substituted with 1, 2 or 3 Rₐ;
R₄, R₅, R₆, and R₇ are each independently selected from H, F, Cl, Br, I and C₁₋₃ alkyl, wherein the C₁₋₃ alkyl is optionally substituted with 1, 2 or 3 R_{c};
n is 0 or 1;
m is 1 or 2;
ring A is selected from pyrazolyl and tetrahydropyranyl, wherein the pyrazolyl and tetrahydropyranyl are optionally substituted with 1, 2 or 3 R_{d};
Rₐ and R_{c} are each independently selected from D, F, Cl, Br and I;
R_{d} is selected from F, Cl, Br, I, C₁₋₃ alkyl and C₁₋₃ alkoxy, wherein the C₁₋₃ alkyl and C₁₋₃ alkoxy are optionally substituted with 1, 2 or 3 R;
R is selected from F, Cl, Br and I.

The present disclosure provides a compound represented by formula (I) or a pharmaceutically acceptable salt thereof, 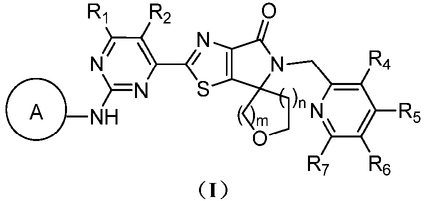 wherein
R₁ and R₂ are each independently selected from H and C₁₋₃ alkyl, wherein the C₁₋₃ alkyl is optionally substituted with 1, 2 or 3 Rₐ;
R₄, R₅, R₆, and R₇ are each independently selected from H, F, Cl, Br, I and C₁₋₃ alkyl, wherein the C₁₋₃ alkyl is optionally substituted with 1, 2 or 3 R_{c};
n is 0 or 1;
m is 1 or 2;
ring A is selected from pyrazolyl and tetrahydropyranyl, wherein the pyrazolyl and tetrahydropyranyl are optionally substituted with 1, 2 or 3 R_{d};
Rₐ and R_{c} are each independently selected from D, F, Cl, Br and I;
R_{d} is selected from F, Cl, Br, I, C₁₋₃ alkyl and C₁₋₃ alkoxy, wherein the C₁₋₃ alkyl and C₁₋₃ alkoxy are optionally substituted with 1, 2 or 3 R;
R is selected from F, Cl and Br.

In some embodiments of the present disclosure, the above-mentioned R₁ and R₂ are each independently selected from H, CH₃ and CH₂CH₃, wherein the CH₃ and CH₂CH₃ are optionally substituted with 1, 2 or 3 Rₐ, and other variables are as defined in the present disclosure.

In some embodiments of the present disclosure, the above-mentioned R₁ and R₂ are each independently selected from H, CH₃, CHF₂, CD₃ and CH₂CH₃, and other variables are as defined in the present disclosure.

In some embodiments of the present disclosure, the above-mentioned R₄, R₅, R₆, and R₇ are each independently selected from H, F, Cl, Br, I and CH₃, wherein the CH₃ is optionally substituted with 1, 2 or 3 R_{c}, and other variables are as defined in the present disclosure.

In some embodiments of the present disclosure, the above-mentioned R₄, R₅, R₆, and R₇ are each independently selected from H, F, Cl, Br, I and CH₃, and other variables are as defined in the present disclosure.

In some embodiments of the present disclosure, the above-mentioned R_{d} is selected from F, Cl, Br, I, CH₃ and OCH₃, wherein the CH₃ and OCH₃ are optionally substituted with 1, 2 or 3 R, and other variables are as defined in the present disclosure.

In some embodiments of the present disclosure, the above-mentioned R_{d} is selected from CH₃ and OCH₃, and other variables are as defined in the present disclosure.

In some embodiments of the present disclosure, the above-mentioned ring A is selected from 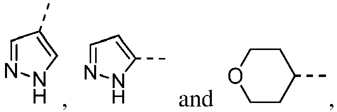 wherein the 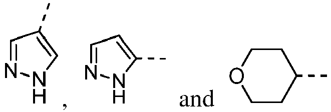 are optionally substituted with 1, 2 or 3 R_{d}, and other variables are as defined in the present disclosure.

In some embodiments of the present disclosure, the above-mentioned ring A is selected from 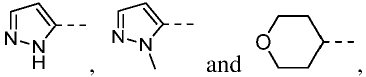 and other variables are as defined in the present disclosure.

In some embodiments of the present disclosure, the above-mentioned structural moiety 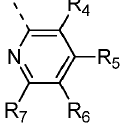 is selected from 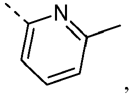 and other variables are as defined in the present disclosure.

The present disclosure also includes some embodiments that are obtained by combining any of the above-mentioned variables.

In some embodiments of the present disclosure, the above-mentioned compound or a pharmaceutically acceptable salt thereof is disclosed, wherein the compound is selected from: 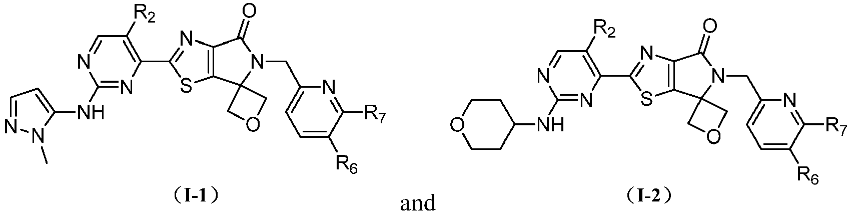 wherein
R₂, R₆ and R₇ are as defined in the present disclosure.

The present disclosure also provides a compound represented by the following formula or a pharmaceutically acceptable salt thereof,

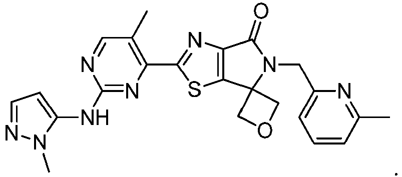

The present disclosure also provides use of the above-mentioned compound or a pharmaceutically acceptable salt thereof in the manufacture of a medicament for treating a solid tumor.

The present disclosure provides crystal form A of WX001, which is characterized by an X-ray powder diffraction pattern having characteristic diffraction peaks at 2θ angles of: 10.2080±0.2000°, 18.8429±0.2000°, 20.6217±0.2000°;

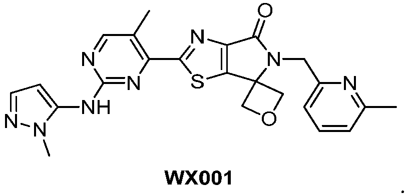

In some embodiments of the present disclosure, the X-ray powder diffraction pattern of the above-mentioned crystal form A, expressed in 2θ angle, comprises at least 4 or 5 diffraction peaks selected from: 10.2080±0.2000°, 18.8429±0.2000°, 20.6217±0.2000°, 25.0767±0.2000°, and 25.4797±0.2000°.

In some embodiments of the present disclosure, the X-ray powder diffraction pattern of the above-mentioned crystal form A has characteristic diffraction peaks at 2θ angles of: 10.2080±0.2000°, 18.8429±0.2000°, 20.6217±0.2000°, 25.0767±0.2000°, 25.4797±0.2000°.

In some embodiments of the present disclosure, the X-ray powder diffraction pattern of the above-mentioned crystal form A, expressed in 2θ angle, comprises at least 6, 7 or 8 diffraction peaks selected from: 10.2080±0.2000°, 15.2687±0.2000°, 17.6747±0.2000°, 18.8429±0.2000°, 20.6217±0.2000°, 21.0531±0.2000°, 25.0767±0.2000°, and 25.4797±0.2000°.

In some embodiments of the present disclosure, the X-ray powder diffraction pattern of the above-mentioned crystal form A has characteristic diffraction peaks at 2θ angles of: 10.2080±0.2000°, 15.2687±0.2000°, 17.6747±0.2000°, 18.8429±0.2000°, 20.6217±0.2000°, 21.0531±0.2000°, 25.0767±0.2000°, 25.4797±0.2000°.

In some embodiments of the present disclosure, the X-ray powder diffraction pattern of the above-mentioned crystal form A, expressed in 2θ angle, comprises at least 9, 10, 11 or 12 diffraction peaks selected from: 10.2080±0.2000°, 14.4684±0.2000°, 15.2687±0.2000°, 17.6747±0.2000°, 18.8429±0.2000°, 20.6217±0.2000°, 21.0531±0.2000°, 21.5713±0.2000°, 22.0420±0.2000°, 22.4540±0.2000°, 25.0767±0.2000°, and 25.4797±0.2000°.

In some embodiments of the present disclosure, the X-ray powder diffraction pattern of the above-mentioned crystal form A has characteristic diffraction peaks at 2θ angles of: 10.2080±0.2000°, 14.4684±0.2000°, 15.2687±0.2000°, 17.6747±0.2000°, 18.8429±0.2000°, 20.6217±0.2000°, 21.0531±0.2000°, 21.5713±0.2000°, 22.0420±0.2000°, 22.4540±0.2000°, 25.0767±0.2000°, 25.4797±0.2000°.

In some embodiments of the present disclosure, the X-ray powder diffraction pattern of the above-mentioned crystal form A has characteristic diffraction peaks at 2θ angles of: 10.2080±0.2000°, 18.8429±0.2000°, and/or 20.6217±0.2000°, and/or 9.4003±0.2000°, and/or 10.4856±0.2000°, and/or 14.4684±0.2000°, and/or 15.0133±0.2000°, and/or 15.2687±0.2000°, and/or 15.6003±0.2000°, and/or 15.9518±0.2000°, and/or 16.6214±0.2000°, and/or 17.6747±0.2000°, and/or 17.9514±0.2000°, and/or 18.4703±0.2000°, and/or 19.1531±0.2000°, and/or 19.6571±0.2000°, and/or 21.0531±0.2000°, and/or 21.2894±0.2000°, and/or 21.5713±0.2000°, and/or 22.0420±0.2000°, and/or 22.4540±0.2000°, and/or 23.1098±0.2000°, and/or 24.5027±0.2000°, and/or 25.0767±0.2000°, and/or 25.4797±0.2000°, and/or 25.8919±0.2000°, and/or 26.3255±0.2000°, and/or 26.9544±0.2000°, and/or 28.3997±0.2000°, and/or 29.0345±0.2000°, and/or 29.3507±0.2000°, and/or 33.5390±0.2000°, and/or 34.2457±0.2000°, and/or 37.9776±0.2000°.

In some embodiments of the present disclosure, the X-ray powder diffraction pattern of the above-mentioned crystal form A has characteristic diffraction peaks at 2θ angles of: 10.2080°, 10.4856°, 14.4684°, 15.0133°, 15.2687°, 15.9518°, 16.6214°, 17.6747°, 17.9514°, 18.4703°, 18.8429°, 19.1531°, 20.6217°, 21.0531°, 21.2894°, 21.5713°, 22.0420°, 22.4540°, 25.0767°, 25.4797°, 26.3255°, 26.9544°.

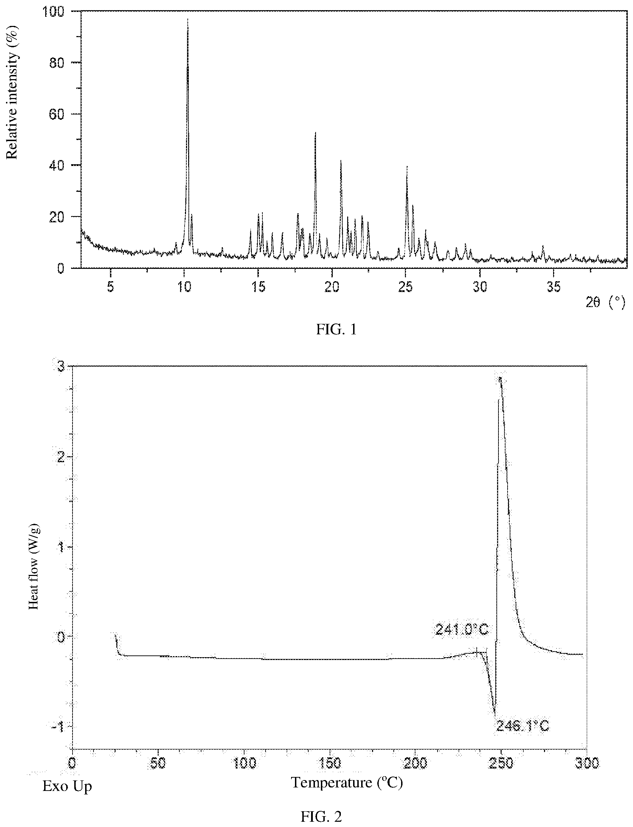

In some embodiments of the present disclosure, the XRPD pattern of the above-mentioned crystal form A is substantially as shown in FIG. 1.

In some embodiments of the present disclosure, the analysis data of the XRPD pattern of the above-mentioned crystal form A is as shown in Table 1:

**Table 1 The analysis data of the XRPD pattern of the crystal form A of WX001**

| **No.** | **2θ Angle (°)** | **D-spacing (Å)** | **Relative intensity (%)** | **No.** | **2θ Angle** | **D-spacing** | **Relative intensity** |
|---|---|---|---|---|---|---|---|
| 1 | 9.4003 | 9.41 | 4.31 | 18 | 21.2894 | 4.17 | 12.24 |
| 2 | 10.2080 | 8.67 | 100.00 | 19 | 21.5713 | 4.12 | 16.68 |
| 3 | 10.4856 | 8.44 | 18.12 | 20 | 22.0420 | 4.03 | 18.34 |
| 4 | 14.4684 | 6.12 | 12.63 | 21 | 22.4540 | 3.96 | 15.00 |
| 5 | 15.0133 | 5.90 | 18.36 | 22 | 23.1098 | 3.85 | 2.96 |
| 6 | 15.2687 | 5.80 | 19.23 | 23 | 24.5027 | 3.63 | 4.08 |
| 7 | 15.6003 | 5.68 | 7.48 | 24 | 25.0767 | 3.55 | 37.76 |
| 8 | 15.9518 | 5.56 | 11.70 | 25 | 25.4797 | 3.50 | 23.82 |
| 9 | 16.6214 | 5.33 | 11.12 | 26 | 25.8919 | 3.44 | 9.21 |
| 10 | 17.6747 | 5.02 | 19.86 | 27 | 26.3255 | 3.39 | 12.81 |
| 11 | 17.9514 | 4.94 | 10.14 | 28 | 26.9544 | 3.31 | 7.34 |
| 12 | 18.4703 | 4.80 | 10.70 | 29 | 28.3997 | 3.14 | 5.21 |
| 13 | 18.8429 | 4.71 | 56.52 | 30 | 29.0345 | 3.08 | 7.36 |
| 14 | 19.1531 | 4.63 | 10.90 | 31 | 29.3507 | 3.04 | 4.04 |
| 15 | 19.6571 | 4.52 | 8.63 | 32 | 33.5390 | 2.67 | 3.43 |
| 16 | 20.6217 | 4.31 | 41.16 | 33 | 34.2457 | 2.62 | 7.39 |
| 17 | 21.0531 | 4.22 | 18.31 | 34 | 37.9776 | 2.37 | 2.33 |

In some embodiments of the present disclosure, the above-mentioned crystal form A has a differential scanning calorimetry curve having an onset of an endothermic peak at 241.0±3.0°C.

In some embodiments of the present disclosure, the DSC curve of the above-mentioned crystal form A is as shown in FIG. 2.

In some embodiments of the present disclosure, the above-mentioned crystal form A has a thermogravimetric analysis curve having a weight loss of up to 0.83% at 150.0±3.0°C.

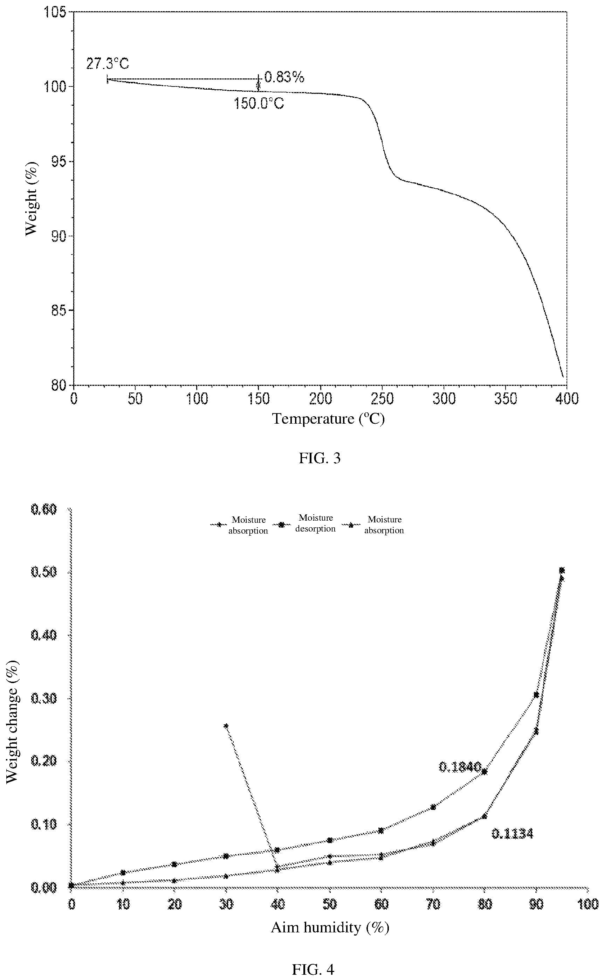

In some embodiments of the present disclosure, the TGA curve of the above-mentioned crystal form A is as shown in FIG. 3.

The present disclosure also provides use of the above-mentioned crystal form A in the manufacture of a medicament for the treatment of a solid tumor.

### Technical effect

The compounds of the present disclosure exhibit superior inhibitory activity against ERK1 and ERK2 enzymes; the compounds of the present disclosure exhibit superior inhibitory activity against HT29 cell proliferation; the compounds of the present disclosure have good solubility under different pH conditions; the compounds of the present disclosure have excellent pharmacokinetic properties and tumor inhibitory effect.

### Definition and term

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Some specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Unless otherwise specified, the term "isomer" is intended to include geometric isomers, cis- or trans- isomers, stereoisomers, enantiomers, optical isomers, diastereomers, and tautomers.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomer, (D)-isomer, (L)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(+)" means dextroisomer, "(-)" means levoisomer, and "(±)" means racemate.

Unless otherwise specified, a wedged solid bond ( 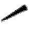 ) and a wedged dashed bond ( 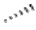 ) indicate the absolute configuration of a stereocenter; a straight solid bond ( 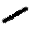 ) and a straight dashed bond ( 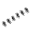 ) indicate the relative configuration of a stereocenter; a wavy line ( 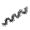 ) indicates a wedged solid bond ( 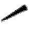 ) or a wedged dashed bond ( 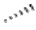 ); or a wavy line ( 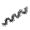 ) indicates a straight solid bond ( 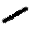 ) and a straight dashed bond ( 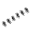 ).

Unless otherwise specified, the term "tautomer" or "tautomeric form" means that different functional groups in an isomer are in dynamic equilibrium and can be rapidly converted into each other at room temperature. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2,4-dione and 4-hydroxypent-3-en-2-one.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (R)- and (S)-isomer, or *D* and *L* isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to afford the pure enantiomer. In addition, the enantiomer and the diastereoisomer are generally isolated through chromatography which uses a chiral stationary phase and optionally combines with a chemical derivative method (for example, carbamate generated from amine).

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When the number of a substituent is 0, it means that the substituent does not exist. For example, -A-(R)₀ means that the structure is actually -A.

When a substituent is vacant, it means that the substituent does not exist. For example, when X is vacant in A-X, the structure of A-X is actually A.

When one of variables is a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, such substituent can be bonded to any atom on the ring. For example, a structural moiety 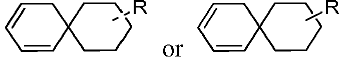 represents the substituent R thereof can be substituted at any site on cyclohexyl or cyclohexadiene. When an enumerated substituent does not indicate through which atom it is linked to the substituted group, such substituent can be bonded through any of its atoms. For example, a pyridyl group as a substituent may be linked to the substituted group through any one of carbon atoms on the pyridine ring.

When an enumerated linking group does not indicate its linking direction, its linking direction is arbitrary. For example, when the linking group L in 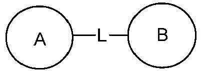 is **-M-W-,** the -M-W- can be linked to the ring A and the ring B in the same direction as the reading order from left to right to constitute 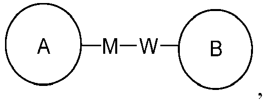 or can be linked to the ring A and the ring B in the reverse direction as the reading order from left to right to constitute 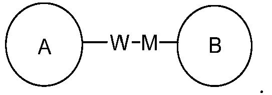 A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond ( 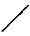 ), a straight dashed bond ( 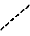 ), or a wavy line ( 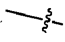 ). For example, the straight solid bond in -OCH₃ indicates that the group is connected to other groups through the oxygen atom in the group; the straight dashed bond in 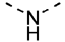 indicates that the group is connected to other groups through two ends of the nitrogen atom in the group; the wavy line in 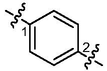 indicates that the group is connected to other groups through the 1- and 2-carbon atoms in the phenyl group; 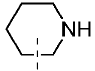 indicates that any connectable site on the piperidinyl group can be connected to other groups through one chemical bond, including at least four connection ways, 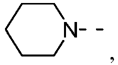 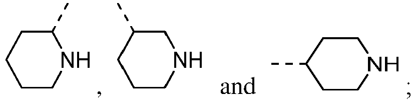 even if a H atom is drawn on -N-, 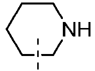 still includes the connection way of 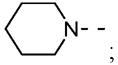 it's just that when one chemical bond is connected, the H at this site will be reduced by one, and the group will become the corresponding monovalent piperidinyl group.

Unless otherwise specified, the number of atoms on a ring is generally defined as the number of ring members, e.g., "5-7 membered ring" refers to a "ring" of 5-7 atoms arranged circumferentially.

Unless otherwise specified, the term "C₁₋₃ alkyl" is used to indicate a linear or branched saturated hydrocarbon group consisting of 1 to 3 carbon atoms. The C₁₋₃ alkyl group includes C₁₋₂ and C₂₋₃ alkyl groups and the like. It may be monovalent (e.g., methyl), divalent (e.g., methylene) or multivalent (e.g., methenyl). Examples of C₁₋₃ alkyl groups include, but are not limited to, methyl (Me), ethyl (Et), propyl (including n-propyl and isopropyl), and the like.

Unless otherwise specified, the term "C₁₋₃ alkoxy" refers to an alkyl group containing 1 to 3 carbon atoms and attached to the remainder of a molecule by an oxygen atom. The C₁₋₃ alkoxy group includes C₁₋₂, C₂₋₃, C₃ and C₂ alkoxy groups, and the like. Examples of C₁₋₃ alkoxy groups include, but are not limited to, methoxy, ethoxy, propoxy (including n-propoxy and isopropoxy), and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to examples disclosed herein.

Throughout this specification, a reference to "an embodiment" or "embodiments" or "in another embodiment" or "in some embodiments" means that a specifically referenced element, structure, or characteristic described in connection with that embodiment is included in at least one embodiment. Accordingly, a phrase "in one embodiment" or "in an embodiment" or "in another embodiment" or "in some embodiments" appearing in various places throughout this specification is not necessarily all referring to the same embodiment. Furthermore, a specific element, structure, or characteristic may be combined in any suitable manner in one or more embodiments.

In the present disclosure, Exo Up in a DSC curve indicates upward exotherm.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In a single crystal X-Ray diffraction (SXRD), the diffraction intensity data of a cultivated single crystal are collected using a Bruker D8 venture diffractometer with a light source of CuKα radiation in a scanning mode of φ/ω scan; after collecting the relevant data, the crystal structure is further analyzed by the direct method (Shelxs97) to confirm the absolute configuration.

The present disclosure will be described in detail through examples below. These examples do not mean any limitation to the present disclosure.

All solvents used in the present disclosure are commercially available and can be used without further purification.

Solvents used in the present disclosure are commercially available.

The following abbreviations are used in the present disclosure: aq represents aqueous; eq represents equivalent or equivalence; DCM represents dichloromethane; PE represents petroleum ether; DMSO represents dimethyl sulfoxide; EtOAc represents ethyl acetate; EtOH represents ethanol; MeOH represents methanol; BOC represents tert-butoxycarbonyl, which is an amine protecting group; r.t. represents room temperature; O/N represents overnight; THF represents tetrahydrofuran; Boc₂O represents di-tert-butyl dicarbonate; TFA represents trifluoroacetic acid; DIPEA represents diisopropylethylamine; iPrOH represents 2-propanol; mp represents melting point.

Compounds are named according to general naming principles in the art or by ChemDraw^{®} software, and commercially available compounds are named with their vendor directory names.

### X-ray powder diffractometer (XRPD) method used in the present disclosure

### Instrument model: X'Pert³ X-ray diffractometer of PANalytical

Test method: About 10 mg of sample was used for XRPD detection.

**Table 2 Parameters for XRPD test**

| Parameters | Settings |
|---|---|
| Model | X'Pert³ |
| X-Ray | Cu, kα, Kα1(Å): 1.540598; Kα2 (Å): 1.544426; Kα2/Kα1: 0.50 |
| X-Ray tube setting | 45 kV, 40 mA |
| Divergence slit | 1/8° |
| Scan mode | Continuous |
| Scan range (°2Theta) | 3~40 |
| Scan step time (s) | 46.7 |
| Step size (°2Theta) | 0.0263 |
| Test time (min) | About 5 minutes |

### Differential Scanning Calorimeter (DSC) method used in the present disclosure

### Instrument model: TA 2500 differential scanning calorimeter

**Table 3 Instrument parameters and test methods for DSC**

| Parameters | Settings |
|---|---|
| Method | linear heating |
| Sample pan | Aluminum pan, crimped/open |
| Temperature range | 25 °C to set end temperature |
| Scan rate (°C/min) | 10 |
| Protective gas | N₂ |

### Thermal Gravimetric Analyzer (TGA) method used in the present disclosure

### Instrument model: TA 5500 thermo gravimetric analyzer

**Table 4 Instrument parameters and test methods for TGA**

| Parameters | Settings |
|---|---|
| Method | linear heating |
| Sample pan | Aluminum pan, open |
| Temperature range | RT to set end temperature |
| Scan rate (°C/min) | 10 |
| Protective gas | N₂ |

### Dynamic vapor sorption (DVS) method used in the present disclosure

Dynamic vapor adsorption (DVS) curves were collected on DVS Intrinsic plus of Surface Measurement Systems (SMS) company. The relative humidity at 25 °C was corrected with the deliquescent points of LiCl, Mg(NO₃)₂ and KCl.

**Table 5 DVS test parameters**

| Parameters | Settings |
|---|---|
| Temperature | 25°C |
| Sample amount | 10-20 mg |
| Protective gas and flow rate | N₂, 200 mL/min |
| dm/dt | 0.002 %/min |
| Min dm/dt balance time | 10 min |
| Max balance time | 180 min |
| RH test range | 0%RH-95%RH |
| RH gradient | 10% (0%RH-90%RH, 90%RH-0%RH) |
| | 5% (90%RH-95%RH, 95%RH-90%RH) |

**Table 6 Classification of hygroscopicity evaluation**

| **Classification of hygroscopicity** | ΔW% |
|---|---|
| Deliquescence | Absorbing enough water and forming a liquid |
| Very hygroscopic | ΔW%≥15% |
| Hygroscopic | 15%>ΔW%≥2% |
| Slightly hygroscopic | 2%>ΔW%≥0.2% |
| Not or almost not hygroscopic | ΔW% <0.2% |

| | |
|---|---|
| Note: ΔW% represents weight increase of a test sample by moisture absorption at 25 ± 1 °C and 80 ± 2% RH. | |

### BRIEF DESCRIPTION OF THE DRAWINGS

FIG. 1: the XRPD pattern of the crystal form A of WX001 using Cu-Kα radiation;
FIG. 2: the DSC curve of the crystal form A of WX001;
FIG. 3: the TGA curve of the crystal form A of WX001;
FIG. 4: the DVS pattern of the crystal form A of WX001;
FIG. 5: Tumor growth curves of human melanoma A375 in model animal after administration of solvent and WX001 respectively;
FIG. 6: Rate of weight change in model animal of human melanoma A375 during the administration.

### DETAILED DESCRIPTION OF THE INVENTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Reference example 1

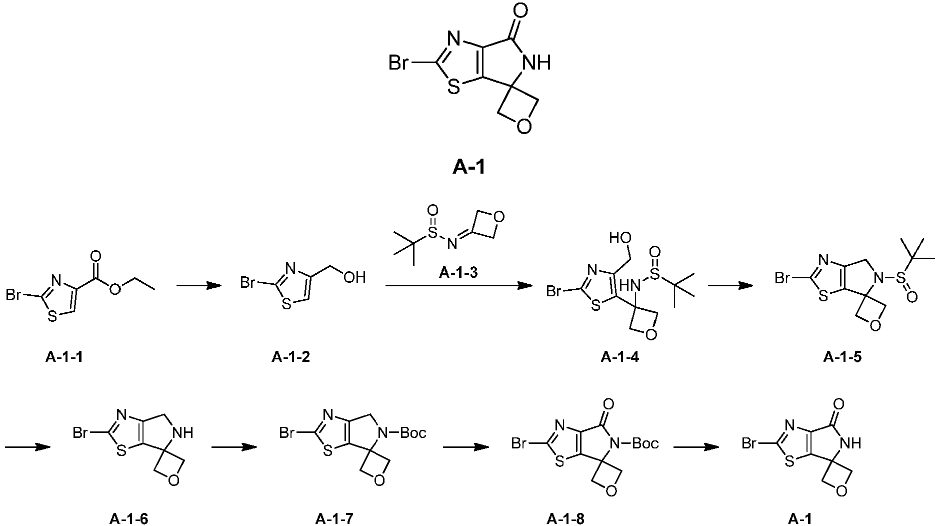

### Step 1: Synthesis of compound A-1-2.

To a reaction flask were added **A-1-1** (150 g, 635.36 mmol, 1 *eq),* calcium chloride (70.51 g, 635.36 mmol, 1 *eq),* tetrahydrofuran (500 mL) and ethanol (1000 mL). Sodium borohydride (48.07 g, 1.27 mol, 2 *eq*) was added under nitrogen, and the mixture was reacted at 20°C for 15 hours. After the reaction was completed, the reaction solution was concentrated under reduced pressure. The concentrate was diluted with 15% citric acid aqueous solution (4000 mL), and extracted with ethyl acetate (4000 mLx3). The organic phases were combined, washed with saturated brine (2000 mL), dried over anhydrous sodium sulfate, and filtered. The filtrate was concentrated under reduced pressure to dryness to give a crude product. The crude product was purified by column chromatography to give **A-1-2.** ¹H NMR (400 MHz, DMSO-*d*₆) δ (ppm) = 7.47 (s, 1H), 5.58 (br s, 1H), 4.52 (s, 2H).

### Step 2: Synthesis of compound A-1-4.

To a reaction flask were added **A-1-2** (102 g, 525.64 mmol, 1 *eq)* and 2-methyltetrahydrofuran (1000 mL). The atmosphere was replaced with nitrogen gas. The mixture was cooled to -70°C, and lithium diisopropylamide (2 M, 525.64 mL, 2.0 *eq*) was slowly added dropwise. The mixture was stirred at -70°C for 30 minutes. Then, a solution of **A-1-3** (138.18 g, 788.46 mmol, 1.5 eq) in 2-methyltetrahydrofuran (400 mL) was slowly added dropwise, and the mixture was reacted at -70°C for another 1 hour. After the reaction was completed, the reaction solution was quenched with saturated aqueous ammonium chloride solution (2000 mL), and extracted with ethyl acetate (2000 mL x 4). The layers were separated. The organic phases were combined, washed with saturated brine (1000 mL), dried over anhydrous sodium sulfate, and filtered. The filtrate was concentrated under reduced pressure to dryness to give a crude product. The crude product was first purified by column, and then purified by slurrying with methyl tert-butyl ether to give **A-1-4.** ¹H NMR (400 MHz, DMSO-*d*₆) δ (ppm) = 6.41 (s, 1H), 5.43 (t, *J =* 5.6 Hz, 1H), 5.00 - 4.84 (m, 4H), 4.38 (d, *J =* 5.6 Hz, 2H), 1.12 (s, 9H).

### Step 3: Synthesis of compound A-1-5.

To a reaction flask were added **A-1-4** (50 g, 135.39 mmol, 1 *eq*), azodicarboxylic acid dipiperidide (40.99 g, 162.47 mmol, 1.2 *eq*) and tetrahydrofuran (500 mL). The atmosphere was replaced with nitrogen gas. The mixture was cooled to 0°C, and a solution of tributylphosphine (32.87 g, 162.47 mmol, 40.09 mL, 1.2 *eq*) in tetrahydrofuran (100 mL) was slowly added dropwise. The mixture was reacted at 0°C for 1 hour. After the reaction was completed, water (500 mL) and saturated brine (500 mL) were added to the reaction solution in sequence. The mixture was extracted with ethyl acetate (500 mL x 2). The layers were separated. The organic phases were combined, washed with saturated brine (300 mL), dried over anhydrous sodium sulfate, and filtered. The filtrate was concentrated under reduced pressure to dryness to give a crude product. The crude product was slurried with 500 mL of methyl tert-butyl ether, and filtered. The filtrate was collected, and concentrated to give a crude product. The crude product was slurried with 50 mL of n-hexane, and filtered. The filter cake was collected and dried to give **A-1-5.** ¹H NMR (400 MHz, DMSO-*d*₆) δ (ppm) = 5.29 (d, *J =* 7.5 Hz, 1H), 4.88 - 4.75 (m, 3H), 4.60 (d, *J =* 12.9 Hz, 1H), 4.22 (d, *J =* 12.9 Hz, 1H), 1.26 (s, 9H).

### Step 4: Synthesis of compound A-1-6.

To a reaction flask were added **A-1-5** (29 g, 82.55 mmol, 1 *eq*), tetrahydrofuran (250 mL) and water (50 mL). The atmosphere was replaced with nitrogen gas. Iodine (2.10 g, 8.26 mmol, 1.66 mL, 0.1 *eq)* was added, and the mixture was reacted at 50°C for 18 hours. Then additional iodine (2.10 g, 8.26 mmol, 1.66 mL, 0.1 *eq*) was added, and the mixture was reacted at 50°C for another 6 hours. After the reaction was completed, a solution of crude **A-1-6** was obtained and used directly in the next step.

### Step 5: Synthesis of compound A-1-7.

To the solution containing crude **A-1-6** was added sodium carbonate (17.50 g, 165.11 mmol, 2 *eq*). The atmosphere was replaced with nitrogen gas. Di-tert-butyl carbonate (27.03 g, 123.83 mmol, 28.45 mL, 1.5 *eq)* was added. The mixture was reacted at 20°C for 12 hours. After the reaction was completed, the reaction solution was poured into water (200 mL), and then extracted with ethyl acetate (300 mL x 3). The layers were separated. The organic phases were combined, washed with saturated brine (300 mL x 3), dried over anhydrous sodium sulfate, and filtered. The filtrate was concentrated under reduced pressure to give a crude product. The crude product was purified by column chromatography to give **A-1-7.** ¹H NMR (400 MHz, DMSO-*d*₆) δ (ppm) = 5.46 (d, *J =* 5.9 Hz, 1H), 5.32 (d, *J =* 6.3 Hz, 1H), 4.64 (d, *J =* 6.1 Hz, 1H), 4.55 (d, *J* = 5.8 Hz, 1H), 4.49 (d, *J* = 9.4 Hz, 2H), 1.53 (s, 9H).

### Step 6: Synthesis of compound A-1-8.

To a reaction flask were added **A-1-7** (26.5 g, 76.32 mmol, 1 *eq),* glacial acetic acid (1.37 g, 22.90 mmol, 1.31 mL, 0.3 *eq)* and acetonitrile (260 mL). The atmosphere was replaced with nitrogen gas. The mixture was heated to 50 °C, and a solution of sodium chlorite (32.48 g, 305.28 mmol, 85% purity, 4 *eq*) in water (70 mL) was added dropwise. After the addition was completed, the mixture was reacted at 50 °C for another 12 hours. Then, additional sodium chlorite (8.97 g, 99.21 mmol, 1.3 *eq*) and glacial acetic acid (458.31 mg, 7.63 mmol, 436.49 µL, 0.1 *eq*) were added, and the mixture was reacted at 50°C for another 6 hours. After the reaction was completed, the reaction solution was quenched with aqueous saturated sodium sulfite solution (150 mL), and water (90 mL) was added. The mixture was left to stand, and the organic phase was separated. The aqueous phase was extracted with ethyl acetate (90 mL). The combined organic phase was washed with saturated brine (90 mL), dried over anhydrous sodium sulfate, and filtered. The filtrate was concentrated to give a crude product. The crude product was slurried with ethyl acetate : n-hexane (1:5, 120 mL) with stirring, and filtered. The filter cake was collected and dried to give **A-1-8.** ¹H NMR (400 MHz, CDCls) δ (ppm) = 5.61 (d, *J* = 6.6 Hz, 2H), 4.76 (d, *J =* 6.6 Hz, 2H), 1.66 (s, 9H).

### Step 7: Synthesis of compound A-1.

To a dried reaction flask were added **A-1-8** (10 g, 27.68 mmol, 1 *eq*) and dichloromethane (100 mL). Trifluoroacetic acid (41.04 g, 359.90 mmol, 26.65 mL, 13 *eq)* was added at 0°C. The mixture was reacted at 0°C for 0.5 hours. After the reaction was completed, the reaction solution was slowly poured into aqueous saturated sodium bicarbonate solution (1000 mL), and adjusted to pH of 7~8. The mixture was extracted with dichloromethane (1000 mL x 3). The layers were separated. The organic phases were combined, dried over anhydrous sodium sulfate, and filtered. The filtrate was concentrated under reduced pressure to give A-1. ¹H NMR (400 MHz, DMSO-*d*₆) δ (ppm) = 9.59 (s, 1H), 4.99-4.78 (m, 4H).

### Reference example 2

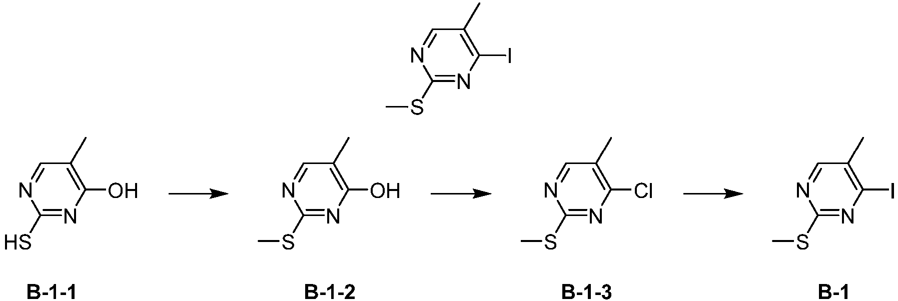

### Step 1: Synthesis of compound B-1-2.

To a reaction flask were added sodium hydroxide (590.8 g, 14.8 mol, 1.05 *eq*), ice water (20 L), and **B-1-1** (2000.00 g, 14.07mol, 1 *eq*). Then methyl iodide (2495.80 g, 17.59 mol, 1.25 eq) was added and the mixture was reacted at 25°C for 2 hours. After the reaction was completed, 6N glacial hydrochloric acid aqueous solution was slowly added into the reaction flask to adjust the pH to 6~7. The mixture was stirred for 0.5 hours, and filtered. The filter cake was collected. Acetonitrile (500 mL) was added to the filter cake. The mixture was stirred for 0.5 hours, and filtered. The filter cake was collected, and dried by baking to give **B-1-2.** ¹H NMR (400 MHz, DMSO-*d*₆) δ (ppm) = 12.69 (br s, 1H), 7.74 (br s, 1H), 2.45 (s, 3H), 1.86 (s, 3H).

### Step 2: Synthesis of compound B-1-3.

To a reaction flask were added acetonitrile (15 L) and **B-1-2** (1500.00 g, 9.60mol, 1 *eq)* at 25°C. Then phosphorus oxychloride (1840.00 g, 12.0 mol, 1.25 eq) was added. The mixture was slowly heated to 62°C, and reacted at 62°C for 12 hours. The reaction solution was poured into water (10.5 L), and solid sodium bicarbonate was added to adjust the pH to 6-7. The mixture was extracted with ethyl acetate (10.5 L), and the layers were separated to give an organic phase. The organic phase was washed with saturated brine (7.5 L), dried over anhydrous sodium sulfate, and filtered. The filtrate was concentrated under reduced pressure to give **B-1-3.** ¹H NMR (400 MHz, DMSO-*d*₆) δ (ppm) = 8.54 (s, 1H), 2.50 (s, 3H), 2.22 (s, 3H).

### Step 3: Synthesis of compound B-1.

To a reaction flask were added **B-1-3** (100 g, 572.57 mmol, 1 *eq*), water (24.76 g, 1.37 mol, 24.76 mL, *2.4eq*) and acetonitrile (1000 mL). The atmosphere was replaced with nitrogen gas. Sodium iodide (571.59 g, 3.81 mol, 6.66 eq) and trimethylchlorosilane (186.61 g, 1.72 mol, 218.00 mL, 3 eq) were added in sequence, and the mixture was reacted at 20°C for 14 hours. After the reaction was completed, dichloromethane (800 mL) and water (1200 mL) were added to the reaction solution in sequence. Then solid sodium bicarbonate was added to adjust the pH to 6-7. The layers were separated, and the aqueous phase was extracted once with dichloromethane (500 mL). The organic phases were combined, washed successively with aqueous saturated sodium sulfite solution (500 mL) and saturated brine (500 mL), dried over anhydrous sodium sulfate, and filtered. The filtrate was concentrated under reduced pressure to give a crude product. n-Heptane (0.5 L) was added to the crude product, and the mixture was stirred for 1 hour. The mixture was filtered, and the filter cake was collected to give **B-1.** ¹H NMR (400 **MHz, DMSO-*d*₆)** δ (ppm) = 8.34 (s, 1H), 2.48 (s, 3H), 2.21 (s, 3H).

### Example 1

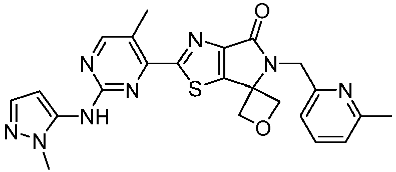

### Synthetic route:

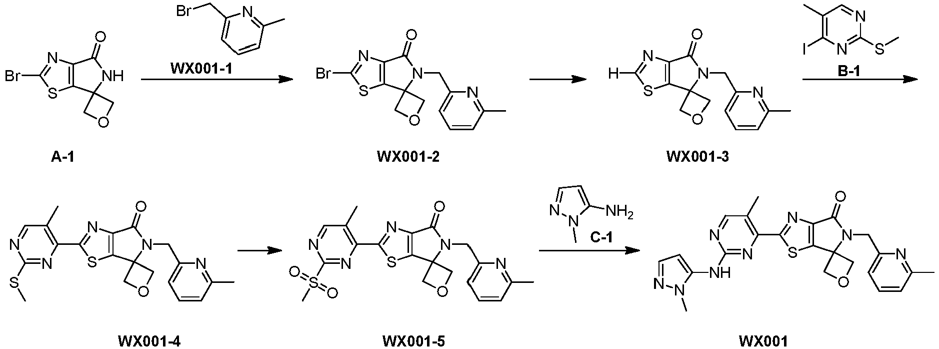

### Step 1: Synthesis of WX001-2

To a reaction flask were added **A-1** (500 mg, 1.92 mmol, 1 *eq*)*,* **WX001-1** (427.54 mg, 2.30 mmol, 1.2 *eq*) and *N'N* - dimethylformamide (3 mL). The atmosphere was replaced with nitrogen gas. Cesium carbonate (935.92 mg, 2.87 mmol, 1.5 *eq)* was added, and the mixture was reacted at 25°C for 16 hours. After the reaction was completed, the reaction solution was poured into water (20 mL), and the mixture was extracted with ethyl acetate (30 mL x 3). The layers were separated. The organic phases were combined, washed with saturated brine (30 mL x 3), dried over anhydrous sodium sulfate, and filtered. The filtrate was concentrated under reduced pressure to give a crude product. The crude product was purified by thin layer chromatography on silica gel plate to give **WX001-2.** LCMS (m/z): 366, 368 [M+H]⁺.

### Step 2: Synthesis of WX001-3

In a pre-dried reaction flask, the atmosphere was replaced with nitrogen, and wet palladium on carbon (0.1 g, 819.15 µmol, 10% purity, 1 *eq)* and ethanol (20 mL) were added. Then **WX001-2** (300 mg, 819.15 µmol, 1 *eq)* was added. The atmosphere was replaced with nitrogen three times, and the mixture was stirred to react at 50°C and 50 Psi for 24 hours. After the reaction was completed, the reaction solution was filtered, and the filtrate was concentrated under reduced pressure to give a crude product. The crude product was purified by thin layer chromatography on silica gel plate to give **WX001-3.** ¹H NMR (400 MHz, CDCl₃) δ (ppm) = 8.96 (s, 1H), 7.58 (t, *J* = 7.6 Hz, 1H), 7.19 (d, *J* = 7.6 Hz, 1H), 7.09 (d, *J =* 7.5 Hz, 1H), 5.26 (d, *J* = 7.6 Hz, 2H), 5.14 (s, 2H), 4.80 (d, *J* = 7.6 Hz, 2H), 2.55 (s, 3H).

### Step 3: Synthesis of WX001-4

To a dried reaction flask were added **WX001-3** (60 mg, 208.81 µmol, 1 *eq*), tetrahydrofuran (1 mL) and zinc chloride solution (0.7 M, 298.31 µL, 1 *eq*). The mixture was coolded to -78°C, and lithium hexamethyldisilazide (1 M, 417.63 µL, 2 *eq*) was added. The mixture was reacted at 20°C for 1 hour to give reaction solution 1.

A mixture of **B-1** (55.57 mg, 208.81 µmol, 1 *eq)* and tetrakis(triphenylphosphine)palladium (7.24 mg, 6.26 µmol, 0.03 *eq)* in *N'N-*dimethylacetamide (1 mL) was heated to 50°C under nitrogen, and then reaction solution 1 was added dropwise. After the addition was completed, the mixture was reacted at 50°C for another 1 hour. After the reaction was completed, the reaction solution was poured into water (5 mL), and then extracted with dichloromethane (30 mL x 3). The layers were separated. The organic phases were combined, washed with saturated brine (30 mL x 3), dried over anhydrous sodium sulfate, and filtered. The filtrate was concentrated under reduced pressure to give a crude product. The crude product was purified by thin layer chromatography on silica gel plate to give **WX001-4.** LCMS (m/z): 426.0 [M+H]⁺.

### Step 4: Synthesis of WX001-5

To a reaction flask were added **WX001-4** (100 mg, 235.00 µmol, 1 *eq),* acetonitrile (1 mL) and water (0.5 mL). The atmosphere was replaced with nitrogen gas three times, and potassium monopersulfate (288.95 mg, 470.01 µmol, 2 *eq*) was added. The mixture was reacted at 20°C for 14 hours. After the reaction was completed, the reaction solution was poured into a saturated sodium thiosulfate aqueous solution (5 mL), and the mixture was extracted with dichloromethane (30 mL x 3). The layers were separated. The organic phases were combined, washed successively with saturated aqueous sodium bicarbonate solution (20 mL) and saturated brine (30 mL x 3), dried over anhydrous sodium sulfate, and filtered. The filtrate was concentrated under reduced pressure to give a crude product. The crude product was purified by thin layer chromatography on silica gel plate to give **WX001-5.** LCMS (m/z): 458.0 [M+H]⁺.

### Step 5: Synthesis of WX001

To a dried reaction flask were added **WX001-5** (40 mg, 87.43 µmol, 1 *eq*)*,* **C-1** (16.98 mg, 174.85 µmol, 2 *eq*) and tetrahydrofuran (0.5 mL). The atmosphere was replaced with nitrogen gas. The mixture was cooled to 0°C, and lithium hexamethyldisilazide (1 M, 166.11 µL, 1.9 *eq)* was added dropwise. The mixture was reacted at 0°C for 1 hour. After the reaction was completed, the reaction solution was poured into water (5 mL), and the mixture was extracted with dichloromethane (30 mL x 3). The layers were separated. The organic phases were combined, washed with saturated brine (30 mL x 3), dried over anhydrous sodium sulfate, and filtered. The filtrate was concentrated under reduced pressure to give a crude product. The crude product was purified by preparative high performance liquid phase chromatography (column: Waters Xbridge BEH C18 100*25mm*5µm; mobile phase: [water (10mM ammonium bicarbonate)-acetonitrile]; B (acetonitrile)%: 20%-50%, 10 min) to give **WX001.** ¹H NMR (400 MHz, DMSO-*d*₆) δ = 9.67 (br s, 1H), 8.62 (s, 1H), 7.64 (t, *J* = 7.7 Hz, 1H), 7.43 (d, *J* = 1.8 Hz, 1H), 7.14 (dd, *J* = 2.9, 7.7 Hz, 2H), 6.37 (d, *J* = 1.5 Hz, 1H), 5.12 (d, *J* = 7.2 Hz, 2H), 5.02 (s, 2H), 4.85 (d, *J* = 7.2 Hz, 2H), 3.74 (s, 3H), 2.57 (s, 3H), 2.42 (s, 3H); LCMS (m/z): 475.0 [M+H]⁺.

### Example 2: Preparation of the crystal form A of WX001

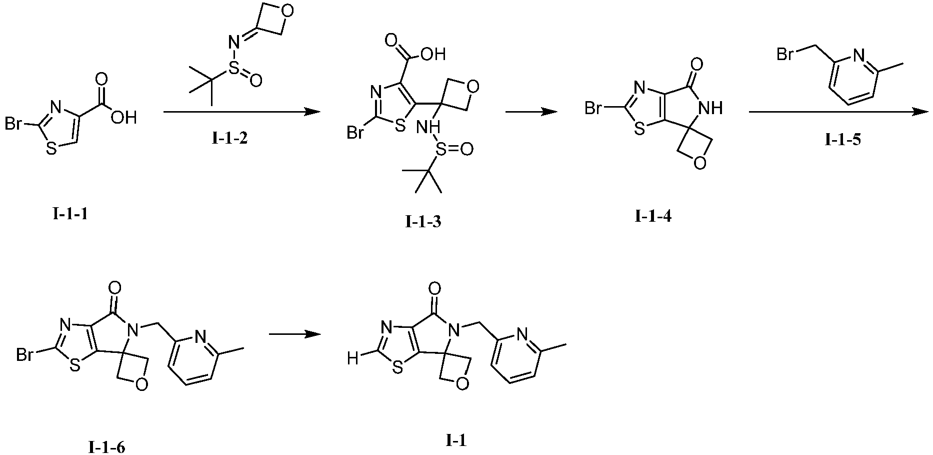

### Step 1: Synthesis of compound I-1-3.

Tetrahydrofuran (12 L) and **I-1-1** (1200 g, 5.69 mol) were added into a reaction kettle. Tetramethylethylenediamine (661.59 g, 5.69 mol) was slowly added into the reaction kettle. The atmosphere was replaced with nitrogen gas. The mixture was cooled to -70 °C (internal temperature). Lithium diisopropylamide (2 M, 6.83 L) was slowly added dropwise, and the mixture was stirred at -70°C for 0.5 hours. A solution of **I-1-2** (1.76 kg, 9.39 mol) in tetrahydrofuran (4.8 L) was slowly added dropwise (1.5 hours), and the mixture was reacted at -70°C for 1 hour. After the reaction was completed, water (6 L) was added dropwise to the reaction solution to quench the reaction mixture. The reaction bottle was washed with water (2.4 L). The mixture was combined, and stirred. The mixture was allowed to warm up to10°C. The layers were separated, and the aqueous phase was extracted with ethyl acetate (6 L). The remaining aqueous phase was adjusted to pH 3~4 with potassium bisulfate aqueous solution (15.6 L), and then extracted three times with ethyl acetate (6 L). The organic phases were combined and washed with saturated brine (6 L). The organic phase was dried with 1200 g of anhydrous sodium sulfate (m/m=1:1), and concentrated under reduced pressure at 45°C to give a crude product. Dichloromethane (2.4 L) and isopropyl ether (7.2 L) were added to the crude product and the mixture was stirred at room temperature for half an hour. The mixture was filtered and the filter cake was collected to give **I-1-3.** ¹H NMR (400 MHz, DMSO-*d*₆) δ = 13.18 (br s, 1H), 6.20 (br s, 1H), 4.93 - 4.82 (m, 4H), 1.09 (s, 9H).

### Step 2: Synthesis of compound I-1-4.

Dichloromethane (1330 mL), **I-1-3** (1330 g, 1.99 mol, crude product), and 4-dimethylaminopyridine (41.37 g, 338.61 mmol) were added to the reaction kettle in sequence. N,N'-carbonyldiimidazole (419.86 g, 2.59 mol) was added in batches, and the mixture was reacted at 50°C for 36 hours. After the reaction was completed, 2 N hydrochloric acid aqueous solution (5.32 L) was added to the reaction solution to adjust the pH to 3-4. The mixture was stirred for 0.5 hours, and then water (5.32 L) was added. The mixture was concentrated to remove the organic solvent. The residue was filtered to give a filter cake. The filter cake was stirred with sodium bicarbonate aqueous solution (5.32 L) for 0.5 hours, then filtered, and washed with water (2.66 L). The filter cake was collected. Absolute ethanol (10.64 L) was added to the crude product. The mixture was stirred for 2 hours, and filtered. The filter cake was washed with absolute ethanol (1.3 L). The filter cake was collected and dried to give **I-1-4.** ¹H NMR (400 MHz, DMSO-*d*₆) δ = 9.58 (br s, 1H), 4.94 - 4.84 (m, 4H).

### Step 3: Synthesis of compound I-1-6.

To a reaction flask were added **I-1-4** (283 g, 1.03 mol), cesium carbonate (505.36 g, 1.55 mol) and N,N'-dimethylformamide (2800 mL). The atmosphere was replaced with nitrogen gas. **I-1-5** (221.24 g, 1.19 mol) was added, and the mixture was reacted at 20 °C for 12 hours. After the reaction was completed, the reaction solution was slowly poured into ice water (14 L). The mixture was stirred for 1 hour, and filtered. The filter cake was collected. Methyl tert-butyl ether (5 L) was added to the crude product and the mixture was stirred for 2 hours, and filtered. The filter cake was collected, and dried to give **I-1-6.** ¹H NMR (400 MHz, DMSO-*d*₆) δ = 7.63 (t, J = 7.7 Hz, 1H), 7.13 (dd, J = 2.8, 7.7 Hz, 2H), 5.04 (d, J = 7.4 Hz, 2H), 4.98 (s, 2H), 4.86 (d, J = 7.4 Hz, 2H), 2.41 (s, 3H).

### Step 4: Synthesis of compound I-1.

Three reactions were carried out in parallel. To a reaction flask were added **I-1-6** (183 g, 468.60 mmol) and tetrahydrofuran (2745 mL). The atmosphere was replaced with nitrogen gas. Diisopropylethylamine (181.69 g, 1.41 mol) and diethyl phosphite (194.14 g, 1.41 mol) were slowly added dropwise at 20 °C, and the mixture was reacted at 40°C for 16 hours. After the reaction was completed, the reaction solution was diluted with water (915 mL), and the mixture was extracted with dichloromethane (1830 mL*3). The organic phase was washed with saturated brine (915 mL), dried over anhydrous sodium sulfate, and filtered. The filtrate was concentrated at 45°C, and dried to give a crude product. The crude product was added to a mixed solvent of methyl tert-butyl ether and n-hexane (total 1098 mL, volume ratio 1:5), and the mixture was stirred for 1 hour. The mixture was filtered, and the filter cake was collected and dried. The filter cake was added to water (5400 mL), and the mixture was stirred for 1 hour, and filtered. The filter cake was collected, and dried to give **I-1.** ¹H NMR (400 MHz, DMSO-*d*₆) δ = 9.32 (s, 1H), 7.64 (t, J = 7.6 Hz, 1H), 7.13 (t, J = 8.4 Hz, 2H), 5.10 (d, J = 7.4 Hz, 2H), 4.99 (s, 2H), 4.81 (d, J = 7.5 Hz, 2H), 2.42 (s, 3H).

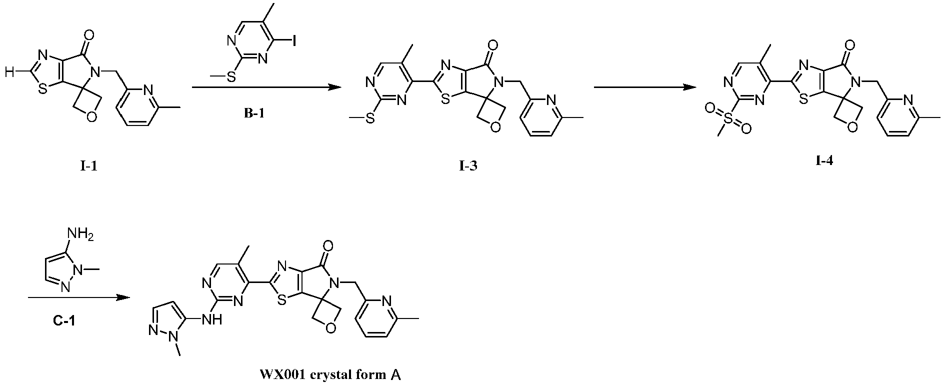

### Step 5: Synthesis of compound I-3.

To two reaction flasks were respectively added a solution of **I-1** (120 g, 403.05 mmol) and zinc chloride (0.7 M, 575.79 mL) in tetrahydrofuran (1200 mL). The atmosphere was replaced with nitrogen gas. The mixture was cooled to 0 °C, and lithium hexamethyldisilazide (1 M, 806.11 mL) was slowly added dropwise. The mixture was allowed to warm up to 20 °C, and then stirred for 1 hour to give reaction solution 1. To another reaction flask were added **B-1** (107.25 g, 403.05 mmol), tetrakis(triphenylphosphine)palladium (13.97 g, 12.09 mmol) and N,N'-dimethylformamide (600 mL), and the reaction solution was heated to 50 °C to give reaction solution 2. Reaction solution 1 was slowly added dropwise into reaction solution 2, and the mixture was reacted at 50°C for 1 hour. After the reaction was completed, the reaction mixture was quenched with 0.1M ethylenediamine tetraacetic acid disodium salt (10800 mL) and stirred for 30 min. n-Heptane (4800 mL) was added and the mixture was stirred for 0.5 hours. The mixture was filtered, and the filter cake was collected and dried to give a crude product. The crude product was slurried with ethanol (7200 mL) at 20°C for 2 hours, and filtered. The filter cake was collected, and dried to give **I-3.** ¹H NMR (400 MHz, CDCl₃) δ = 8.54 (s, 1H), 7.54 (t, J = 7.2 Hz, 1H), 7.19 (d, J = 7.4 Hz, 1H), 7.07 (d, J = 7.3 Hz, 1H), 5.29 (d, J = 7.5 Hz, 2H), 5.11 (s, 2H), 4.84 (d, J = 7.6 Hz, 2H), 2.73 (s, 3H), 2.66 (s, 3H), 2.52 (s, 3H).

### Step 6: Synthesis of compound I-4.

To a reaction flask were added **I-3** (120 g, 282.00 mmol), acetonitrile (110 mL) and water (550 mL). The atmosphere was replaced with nitrogen gas. Potassium monopersulfate (329.40 g, 535.81 mmol) was added, and the mixture was reacted at 30 °C for 12 hours. After the reaction was completed, 200 mL of ice-water mixture was added to the reaction solution. Then saturated sodium bicarbonate aqueous solution and saturated sodium thiosulfate aqueous solution were added (600 mL each), followed by 600 mL of water. The mixture was filtered, and the filter cake was collected and dried to give a crude product. 600 mL of absolute ethanol was added to the crude product, and the mixture was stirred for 1 hour. The mixture was filtered, and the filter cake was collected to give **I-4.** ¹H NMR (400 MHz, CDCl₃) δ = 8.90 (s, 1H), 7.55 (t, J = 7.7 Hz, 1H), 7.20 (d, J = 7.7 Hz, 1H), 7.06 (d, J = 7.7 Hz, 1H), 5.31 (d, J = 7.6 Hz, 2H), 5.11 (s, 2H), 4.84 (d, J = 7.6 Hz, 2H), 3.44 (s, 3H), 2.92 (s, 3H), 2.50 (s, 3H).

### Step 7: Synthesis of the crystal form A of WX001.

To a reaction flask were added **I-4** (47 g, 102.73 mmol), **C-1** (25.94 g, 267.09 mmol), dichloromethane (470 mL) and tetrahydrofuran (470 mL). The atmosphere was replaced with nitrogen gas. Lithium hexamethyldisilazide (1 M, 246.54 mL, 2.4 eq) was added dropwise at - 5°C (controlling the internal temperature at -5-3°C), and the mixture was reacted at 0°C for 0.5 hours. After the reaction was completed, the mixture was quenched with deionized water (470 mL). The mixture was concentrated to remove organic solvent, and filtered. The filter cake was collected. The filter cake was stirred with deionized water (1000 mL) at room temperature for 30 minutes, and filtered. The filter cake was collected. The filter cake was stirred with acetonitrile (1000 mL) at room temperature for 30 min, and filtered. The filter cake was collected to give **the crystal form A of WX001.** ¹H NMR (400 MHz, CDCl₃) δ = 8.44 (s, 1H), 7.60 - 7.50 (m, 2H), 7.18 (d, J = 7.4 Hz, 1H), 7.06 (d, J = 7.6 Hz, 1H), 6.88 (s, 1H), 6.42 (d, J = 1.9 Hz, 1H), 5.27 (d, J = 7.6 Hz, 2H), 5.12 (s, 2H), 4.85 (d, J = 7.6 Hz, 2H), 3.85 (s, 3H), 2.69 (s, 3H), 2.52 (s, 3H). The XRPD pattern of the crystal form A of WX001 is shown in FIG. 1; the DSC curve of the crystal form A of WX001 is shown in FIG. 2; and the TGA curve of the crystal form A of WX001 is shown in FIG.3.

### Example 3: Polymorph screening of WX001

### 1. Gas-solid penetration

About 20 mg of the crystal form A of WX001 in each portion was weighed into a 3-mL vial, and about 4 mL of solvent was added to a 20-mL vial. The 3-mL (open) vial was placed in the 20-mL vial, and the 20-mL vial was sealed. The samples adsorbed the solvent and partially dissolved, or were allowed to stand at room temperature for 7 days, and then the solids were collected and tested by XRPD. The test results are shown in Table 7.

**Table 7 Summary of the gas-solid penetration test**

| **Solvent** | **Test results** |
|---|---|
| MeOH | Crystal form A |
| Acetone | Crystal form A |
| EtOAc | Crystal form A |
| MTBE | Crystal form A |
| ACN | Crystal form A |
| Toluene | Crystal form A |
| 1,4-Dioxane | Crystal form A |
| H₂O | Crystal form A |

### 2. Gas-liquid diffusion

About 20 mg of the crystal form A of WX001 in each portion was weighed into a 3-mL vial, and dissolved in 1.2~1.8 mL of solvent. About 4 mL of anti-solvent was added to a 20-mL vial. The 3-mL (open) vial containing a clear solution was placed in the 20-mL vial, and then the 20-mL vial was sealed and allowed to stand at room temperature. The resulting solid was collected and tested by XRPD. The test results are shown in Table 8.

**Table 8 Summary of the gas-liquid diffusion test**

| **Solvent** | **Anti-solvent** | **Test results** |
|---|---|---|
| CHCl₃ | Acetone | Crystal form A |
| DMSO | EtOAc | Crystal form A* |
| | Toluene | Crystal form A* |

| | | |
|---|---|---|
| * indicates that the solid was obtained by volatilization at room temperature. | | |

### 3. Slow volatilization

15~20 mg of the crystal form A of WX001 was weighed into a 3-mL vial, and dissolved in 1.0~3.0 mL of solvent. The vial was sealed with a sealing film, and 4 pinholes were punched in the sealing film. The solution was left to stand at room temperature to slowly volatilize. The resulting solid was collected and tested by XRPD. The test results are shown in Table 9.

**Table 9 Summary of the slow volatilization test**

| **Solvent (v/v)** | **Test results** |
|---|---|
| MeOH/DCM (1:1) | Crystal form A |
| THF/H₂O (1:1) | Crystal form A |
| CHCl₃ | Crystal form A |
| 1,4-dioxane | Crystal form A |

### 4. Slow cooling

15~35 mg of the crystal form A of WX001 in each portion was weighed into a 3-mL vial, and dissolved in 1.0~3.0 mL of solvent. The solution was stirred and equilibrated at 50 °C for about 3.5 hours, and then filtered. The supernatant was collected. The resulting supernatant was placed in a biochemical incubator, cooled from 50 °C to 5 °C at 0.1 °C/min, and then maintained at a constant temperature of 5 °C. The precipitated solids were collected and tested by XRPD. The test results are shown in Table 10.

**Table 10 Summary of the slow cooling test**

| **Solvent (v/v)** | **Test results** |
|---|---|
| CHCl₃ | Crystal form A* |
| THF/H₂O (1:1) | Crystal form A* |
| ACN/H₂O (1:1) | Crystal form A* |

| | |
|---|---|
| * indicates that the sample was clear at 5 °C and -20 °C and the solid was obtained by volatilization at room temperature. | |

### 5. Stirring with temperature cycle

About 25 mg of the crystal form A of WX001 in each portion was weighed into an HPLC vial, and 0.5 mL of solvent was added respectively. The resulting suspension was subjected to a temperature cycle program (the sample was heated to 50 °C and then cooled to 5 °C at a rate of 0.1 °C/min; then this cycle was repeated; finally, the sample was maintained at 5 °C) with magnetic stirring (1000 rpm). The solids were collected by centrifugation, and tested by XRPD. The test results are shown in Table 11.

**Table 11 Summary of the test of stirring with temperature cycle**

| **Solvent (v/v)** | **Test results** |
|---|---|
| MeOH | Crystal form A |
| MIBK | Crystal form A |
| EtOAc | Crystal form A |
| THF/H₂O (1:1) | Crystal form A |
| ACN/H₂O (1:1) | Crystal form A |
| DMAc/H₂O (1:1) | Crystal form A |

### 6. Stirring of suspension at room temperature

About 25 mg of the crystal form A of WX001 in each portion was weighed into an HPLC vial, and 0.5 mL of solvent was added respectively. The obtained turbid liquid was magnetically stirred (1000 rpm) at room temperature for 3 days. The solids were collected by centrifugation, and tested by XRPD. The test results are shown in Table 12.

**Table 12 Summary of the test of stirring of suspension at room temperature**

| **Solvent (v/v)** | **Test results** |
|---|---|
| EtOH | Crystal form A |
| EtOAc | Crystal form A |
| THF | Crystal form A |
| DCM | Crystal form A |
| n-Heptane | Crystal form A |
| H₂O | Crystal form A |
| EtOH/H₂O (0.97:0.03, a_{w}~0.2) | Crystal form A |
| EtOH/H₂O (0.93:0.07, a_{w}~0.4) | Crystal form A |
| EtOH/H₂O (0.86:0.14, a_{w}~0.6) | Crystal form A |
| EtOH/H₂O (0.71:0.29, a_{w}~0.8) | Crystal form A |
| ACN | Crystal form A |

### 7. Stirring of suspension at 50 °C

About 25 mg of the crystal form A of WX001 in each portion was weighed into an HPLC vial, and 0.5 mL of solvent was added respectively. The obtained suspension was magnetically stirred (1000 rpm) at 50 °C for 3 days. The solids were collected by centrifugation, and tested by XRPD. The test results are shown in Table 13.

**Table 13 Summary of the test of stirring of suspension at 50 °C**

| **Solvent (v/v)** | **Test results** |
|---|---|
| IPA | Crystal form A |
| Acetone/H₂O (1:1) | Crystal form A |
| IPAc | Crystal form A |
| MTBE | Crystal form A |
| 2-MeTHF | Crystal form A |
| 1,4-dioxane | Crystal form A |
| CHCl₃/n-heptane (1:1) | Crystal form A |
| Toluene | Crystal form A |
| DMSO/H₂O (1:1) | Crystal form A |
| ACN | Crystal form A |

### 8. Addition of anti-solvent

About 15 mg of the crystal form A of WX001 was respectively weighed into a 20 mL vial, and 0.7~1.0 mL of solvent was added to completely dissolve the solid. The anti-solvent was added dropwise to the clear solution while stirring (1000 rpm) until solid precipitated or until the total volume of anti-solvent reached 10 mL. Samples without solid precipitation were stirred at 5 °C until solid precipitated. Clear samples were then stirred at -20 °C until solid precipitated. Still clear samples were then volatilized at room temperature. The precipitated solid was separated and tested by XRPD. The results are shown in Table 14.

**Table 14 Summary of the test of addition of anti-solvent**

| **Solvent (v/v)** | **Anti-solvent** | **Test results** |
|---|---|---|
| CHCl₃ | MeOH | Crystal form A* |
| | Acetone | Crystal form A |
| | EtOAc | Crystal form A |
| | MTBE | Crystal form A |
| | 2-MeTHF | Crystal form A |
| | ACN | Crystal form A |
| | n-Heptane | Crystal form A |
| | Toluene | Crystal form A |
| MeOH/DCM (1:1) | MIBK | Crystal form A |
| | IPAc | Crystal form A |
| | MTBE | Crystal form A |
| THF/H₂O (1:1) | H₂O | Crystal form A |
| NMP | H₂O | Crystal form A |
| DMAc | H₂O | Crystal form A |

| | | |
|---|---|---|
| * indicates that the sample was clear at room temperature and the solid was obtained by stirring at 5 °C. | | |

### Example 4: Study on hygroscopicity of the crystal form A of WX001

Materials of the assay:
   SMS DVS Advantage dynamic vapor sorption apparatus
Method of the assay:
   10~30 mg of the crystal form A of WX001 was weighed and put into a DVS sample pan for testing.
Results of the assay:
   The DVS pattern of the crystal form A of WX001 is shown in FIG. 4, ΔW= 0.1134%.
Conclusion of the assay:
   The weight increase of the crystal form A of WX001 by moisture absorption at 25 °C and 80% RH was 0.1134%, indicating that the crystal form A of WX001 has almost no hygroscopicity.

### Example 5: Stability test of the crystal form A of WX001

12 portions of samples of the crystal form A of WX001 were weighed in parallel, and each portion was about 5 mg. The sample was placed at the bottom of an HPLC vial to form a thin layer. The samples were placed in a chamber at a constant temperature and humidity of 60°C/75% RH conditions and in a 92.5% RH desiccator. The bottles were sealed with a sealing film. Some small holes were punched in the sealing film to ensure that the samples were fully in contact with the ambient air. The caps of the sample bottles placed at 60°C under light and light-shielding conditions (samples under light-shielding conditions were wrapped in tin foil) were tighten. The test results are shown in Table 15 below:

**Table 15 Results of the solid stability test of the crystal form A of WX001**

| **Test conditions** | **Conditions for taking points** | **Crystal form** |
|---|---|---|
| 0 day | -- | Crystal form A |
| 60 °C | 5 days | Crystal form A |
| | 10 days | Crystal form A |
| 92.5% RH | 5 days | Crystal form A |
| | 10 days | Crystal form A |
| Visible light^{#} | Reaching illumination of 1.2E+06 Lux·hrs | Crystal form A |
| Control group of light-shielding | Taking points simultaneously with visible light group | Crystal form A |
| Visible light + UV^{#} | Reaching illumination of 200 W·hrs/m² | Crystal form A |
| Control group of light-shielding | Taking points simultaneously with visible light + UV group | Crystal form A |
| 60 °C/75% RH | 1 month | Crystal form A |
| | 2 months | Crystal form A |
| | 3 months | Crystal form A |

| | | |
|---|---|---|
| #: ICH conditions | | |

**Conclusion: the** crystal form A of WX001 has good stability.

### Assay example 1. Assay of in vitro kinase activity

### 1. Purpose of the assay:

The ability of the compound to inhibit ERK1 and ERK2 kinase activity was measured.

### 2. Assay buffer:

20 mM Hepes (pH 7.5), 10 mM MgCl₂, 1 mM ethylenebis(oxyethylenenitrilo)tetraacetic acid (EGTA), 0.02% Brij35, 0.02 mg/mL bovine serum albumin (BSA), 0.1 mM Na₃VO₄, 2 mM dithiothreitol (DTT), 1% DMSO.

### 3. Processing of compound:

The assay compound was dissolved in 100% DMSO to prepare a stock solution of specific concentration. The compound was serially diluted in DMSO solution using Integra Viaflo Assist smart pipette.

### 4. Method of the assay:

1) The substrate MBP was prepared in freshly prepared reaction buffer;
2) ERK1 (or ERK2) kinase was added to the above-mentioned MBP solution and mixed gently;
3) The compound dissolved in 100% DMSO was added to the kinase reaction system using ultrasound technology (Echo550; nanoliter range), and the mixture was incubated at room temperature for 20 minutes;
4) ³³P-ATP (specific concentration of 10 µCi/µL) was added to the reaction system, and the reaction was started at this time;
5) The mixture was incubated at room temperature for 2 hours;
6) The amount of radioactivity was detected by filter-binding method;
7) ERK1 (or ERK2) kinase activity was calculated as the ratio of the remaining kinase activity in the assay sample to the kinase activity of the control group (treated by DMSO). Curve was fitted using Prism (GraphPad software) and IC₅₀ values were calculated.

5. The assay results are shown in Table 16 and Table 17:

**Table 16: Result of ERK1 kinase activity assay**

| **Compound** | **ERK1** |
|---|---|
| | **IC₅₀ (nM)** |
| **WX001** | 1.4 |

Conclusion: The compound of the present disclosure exhibits excellent inhibitory activity against ERK1 kinase.

**Table 17: Result of ERK2 kinase activity assay**

| **Compound** | **ERK2** |
|---|---|
| | **IC₅₀ (nM)** |
| **WX001** | 0.54 |

Conclusion: The compound of the present disclosure exhibits excellent inhibitory activity against ERK2 kinase.

### Assay example 2. Assay of in vitro cell proliferation inhibition

### 1. Purpose of the assay:

The ability of the compound to inhibit the proliferation of HT29 tumor cells was measured.

### 2. Processing of compound:

The assay compound was dissolved in 100% DMSO to prepare 10 mM stock solution.

### 3. Method and step of the assay:

1) UV light of a biological safety cabin was turned on, and 30 minutes were counted down;
2) In a 37 °C water bath, RPMI1640 medium and trypsin were preheated;
3) After completion of the UV irradiation, the biological safety cabin was opened. The preheated medium, trypsin and phosphate buffered saline (PBS), etc. were wiped with alcohol and placed in the biological safety cabin;
4) HT29 cells were removed from the incubator, and the old medium was removed in biological safety cabin. 10 ml of PBS was added. The mixture was shaken gently, and then PBS was removed;
5) 1.5 ml of preheated 0.25% trypsin was added. The culture vessel was shaken horizontally so that the trypsin evenly covered the cells at the bottom, and placed in an incubator for 2 minutes;
6) Cell digestion was stopped with complete medium, and the cell suspension was pipetted to homogeneity and counted;
7) According to the result of cell counting, the density of cell suspension was adjusted to 1500 cells per well, and the cell suspension was seeded at 50 µl per well;
8) The stock solution of the compound was serially diluted in DMSO solution, and the compound was added to the cell plate using Tecan;
9) The compound-added cell plate and CellTiterGlo were equilibrated at room temperature, and 25 microliters of CellTiterGlo was then added to each well. The cell plate was shaken for 1-2 minutes and then allowed to stand for 10 minutes. The signal value was then detected. The data were analyzed using XL-Fit, and the IC₅₀ of each compound was calculated.

4. The assay result is shown in Table 18:

**Table 18: Result of in vitro cell activity assay**

| Compound | HT29 |
|---|---|
| | IC₅₀ (nM) |
| **WX001** | 66 |

Conclusion: The compound of the present disclosure exhibits excellent inhibitory activity on HT29 cell proliferation.

### Assay example 3. In vivo PK study in mice

### 1. Purpose of the assay:

Female BALB/c mice were used as assay animals to determine the blood concentration of the compound and evaluate the pharmacokinetic behavior after a single administration.

### 2. Procedure of the assay:

Four healthy adult female BALB/c mice were selected, wherein 2 mice were in an intravenous injection group and 2 mice were in an oral group. The vehicle in the intravenous injection group was 5% DMSO+95% (20% HP-β-CD). The compound to be assayed was mixed with an appropriate amount of vehicle for intravenous injection, vortexed and sonicated to prepare a clear solution of 0.5 mg/mL. The clear solution was filtered by a microporous membrane, and then ready for use. The vehicle in the oral group was 5% DMSO+95% (20% HP-β-CD). The compound to be assayed was mixed with the vehicle, vortexed and sonicated to prepare a solution of 0.3 mg/mL. Mice were administered 1 mg/kg intravenously or 3 mg/kg orally, and then whole blood was collected for a certain period. Plasma was prepared. The drug concentration was analyzed by LC-MS/MS method, and the pharmacokinetic parameters were calculated by Phoenix WinNonlin software (Pharsight, USA).

Note: DMSO: dimethyl sulfoxide; HP-β-CD: hydroxypropyl-β-cyclodextrin.

3. The assay result is shown in Table 19:

**Table 19: Results of the PK assay of the compound**

| Compound | Cₘₐₓ (nM) | F% | Oral DNAUC (nM.h/mpk) | Vdₛₛ (L/kg) | Cl (mL/min/kg) | T_{1/2} (h) |
|---|---|---|---|---|---|---|
| **WX001** | 4790 | 154 | 2935 | 1.23 | 18.9 | 1.1 |

| | | | | | | |
|---|---|---|---|---|---|---|
| Note: Cₘₐₓ is maximum concentration; F% is oral bioavailability; DNAUC is AUC_{PO}/Dose, AUC_{PO} is oral exposure, and Dose is drug dose; Vdₛₛ is volume of distribution; Cl is clearance rate; T_{1/2} is half-life; and NA is not available. | | | | | | |

Conclusion: The compound of the present disclosure exhibits excellent oral exposure and bioavailability.

### Assay example 4. Solubility study

### 1. Purpose of the assay:

The solubility of the compound was determined to evaluate the solubility property of the compound.

### 2. Assay solution:

1) Buffer A (pH 2.0): 50 mM phosphate buffer, pH 2.0; Buffer B (pH 6.5): 50 mM phosphate buffer, pH 6.5; Buffer C (pH 7.4): 50 mM phosphate buffer, pH 7.4;
2) Preparation of standard solution:
a) 50% acetonitrile solution and 50% buffer solution were mixed to obtain a diluent;
b) 10 mM (10 µL/compound) compound stock solution was added to the diluent (490 µL/compound) to obtain 200 µM detection standard solution;
c) The 200 µM UV detection standard solution was diluted with 10-fold and 200-fold volume of diluents to obtain 20 µM and 1 µM UV standard solutions, respectively;
d) UV standard solutions of 1 µM, 20 µM, and 200 µM were used as standard solutions for solubility assays.

3. Method of the assay:
1) The compound was dissolved in DMSO to prepare a 10 mM stock solution. Amiodarone hydrochloride, carbamazepine, and chloramphenicol were used as controls in the solubility assay;
2) The stock solutions of the assay compound and the control (10 µL each) were put into a 96-well plate, and 490 µL of three different dissolution media (buffer A, B, C) were added respectively. The pH of the corresponding solubility solutions was 2.0, 6.5 and 7.4, respectively. The theoretical maximum concentration of the assay compound is 200 µM with 2% DMSO;
3) The plate was shaken in a shaker at room temperature (25±2°C) at 600 rpm for 24 hours;
4) 200 µL of the solution was pipetted from the 96-well plate, filtered with suction by a vacuum suction filtration device, and transferred into a new 96-well plate as an assay sample;
5) The compound concentration was determined using HPLC-UV. HPLC conditions are as shown in Table 20:

**Table 20: HPLC conditions**

| Assay method | HPLC-UV detection | | |
|---|---|---|---|
| Instrument | Agilent 1200 | | |
| Mobile phase | A: Water + 0.37% trifluoroacetic acid | | |
| | B: Acetonitrile+0.19% trifluoroacetic acid | | |
| Column | Waters Xbridge RP-C18 (2.1×50 mm, 5 µm) | | |

| | Time (min) | B% | Flow rate |
|---|---|---|---|
| Proportion | 0.00 | 5 | (mL/min) |
| | 2.00 | 90 | 1.0 |
| | 2.50 | 90 | 1.0 |
| | 3.01 | 5 | 1.0 |
| | 4.00 | 5 | 1.0 |
| | | | 1.0 |

6) Three UV standard solutions from low concentration to high concentration (1 µM, 20 µM, 200 µM) were injected into HPLC, and then the assay sample of the compound to be assayed was injected;
7) The UV chromatographic peaks were integrated, and the solubility of the sample was calculated.
4. The assay results are shown in Table 21:

**Table 21: Assay results of compound solubility**

| Compound | Solubility at different pH | | |
|---|---|---|---|
| | pH=2.0 | pH=6.5 | pH=7.4 |
| **WX001** | 199.5 µM | 2.94 µM | 2.64 µM |

Conclusion: The compound of the present disclosure has good solubility under different pH conditions.

### Assay example 5. Assay of in vivo efficacy in mouse model of human melanoma A375:

### 1. Purpose of the assay:

The anti-tumor effect of **WX001** was evaluated using a subcutaneous xenograft tumor model of human melanoma A375 cells in nude mouse.

### 2. Assay animal:

Species: mouse
Strain: BALB/c nude mouse
Age: 6-8 weeks old
Gender: female
Body weight: 18-22 grams
Supplier: Vital River Laboratory Animal Technology Co., Ltd.

### 3. Environment for rearing:

Animals were reared in IVC (independent air supply system, and constant temperature and humidity) cages (3 animals per cage) in SPF grade of animal room at a temperature of 20-26°C and a humidity of 40-70%;
Cage: The cage was made of polycarbonate, and had a volume of 375 mm x 215 mm x 180mm. The bedding material was corncob, which was replaced once a week;
Food: Assay animals had free access to food (dry pelleted food sterilized by irradiation) throughout the assay period;
Drinking water: Assay animals had free access to sterilized water;
Cage identification: The animal information card for each cage should indicate the number, gender, strain, date of receipt, assay numbering of administration schedule, group, and start date of the assay of animals in the cage;
Animal identification: Assay animals were identified by ear tags.

### 4. Assay procedure:

1) Assay cells and culture: Human melanoma A375 cells were cultured in monolayer in vitro. The culture conditions were DMEM medium plus 10% fetal bovine serum, and a 37°C 5% CO₂ incubator. Routine digestion with trypsin-EDTA was performed twice a week for passage. When the cell saturation was 80%-90% and the amount reached the requirement, the cells were harvested, counted, and inoculated;
2) Tumor tissue inoculation and grouping: 0.1 mL (5×10⁵) of A375 cells were subcutaneously inoculated into the right armpit of each mouse. When the average tumor volume reached 170 mm³, the animals were randomly divided into 4 groups and the administration was started. The grouping and administration schedule of the assay were shown in Table 22.

**Table 22: Grouping and administration schedule of assay animals**

| Group | Number of animals | Drug | Dosage (mg/kg) | Cycle of administration | Route and frequency of administration |
|---|---|---|---|---|---|
| **1** | 6 | Solvent control | -- | 21 days | Oral administration (PO), twice daily (BID) |
| | | (Vehicle) | | | |
| **2** | 6 | **WX001** | 12.5 | 21 days | Oral administration (PO), twice daily (BID) |
| **3** | 6 | **WX001** | 25 | 21 days | Oral administration (PO), twice daily (BID) |
| **4** | 6 | **WX001** | 50 | 21 days | Oral administration (PO), twice daily (BID) |

3) Daily observation of assay animals: The development of this assay protocol and any modifications were evaluated and approved by the Institutional Animal Care and Use Committee (IACUC). The use and welfare of assay animals were carried out in accordance with the regulations of the Association for Assessment and Accreditation of Laboratory Animal Care (AAALAC). Animals were monitored daily for health and death. Routine examinations included observation of tumor growth and the effects of drug treatment on the animals' daily behavior such as behavioral activities, food and water intake (visual inspection only), weight changes (weight measurements twice a week), appearance signs or other abnormalities. Animal deaths and side effects in each group were recorded based on the number of animals in each group.
4) Formulation of assay compound
a) Vehicle group: 5% DMSO + 95% (20% HP-β-CD).
b) Assay compound group: A quantitative amount of the assay compound was weighed in a formulation bottle. A corresponding volume of DMSO was added and then the mixture was vortexed to obtain a clear solution. A corresponding volume of 20% HP-β-CD was added and then the mixture was vortexed to obtain a homogeneous suspension.

5) Tumor measurement and assay indicator:
a) Tumor diameter was measured twice a week with a vernier caliper. The calculation formula of tumor volume was: TV=1/2×a×b², wherein a and b represent the long and short diameters of tumor, respectively;
b) The tumor-inhibitory efficacy of the compound was evaluated by TGI (%). TGI (%) reflected the inhibition rate of tumor growth. TGI (%) was calculated as follows: TGI (%) = {[1-(average tumor volume at the end of administration of a treatment group - average tumor volume at the beginning of administration of this treatment group)]/(average tumor volume at the end of treatment in a solvent control group - average tumor volume at the beginning of treatment in the solvent control group)}×100%.

### 5. Assay results:

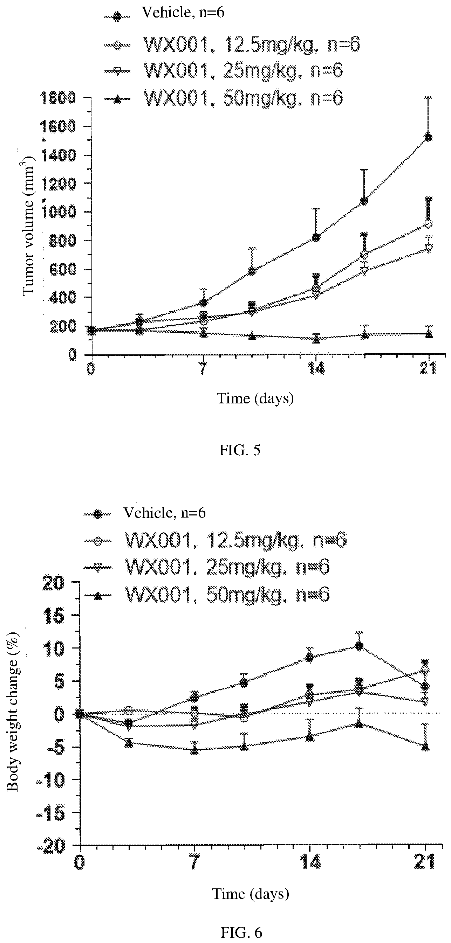

1) As shown in Table 23 and FIG. 5, in the subcutaneous xenograft tumor model of human melanoma A375 cells in nude mouse, **WX001** could inhibit tumor growth in a dose-dependent manner until day 21 after oral administration, at three doses of 12.5 mg/kg, 25 mg/kg and 50 mg/kg, with the TGI of 45%, 58% and 102% respectively.
2) The body weight of assay animals was used as a reference index for indirect determination of drug toxicity. As shown in FIG. 6, when administered to the 21st day, the body weight of all animals in the solvent control group and **WX001** group did not decrease significantly, and the tolerance was good.

**Table 23: Results of in vivo efficacy assay in mouse A375 model**

| Drug | TGI |
|---|---|
| **WX001** (12.5 mg/kg, PO, BID) | 45% |
| **WX001** (25 mg/kg, PO, BID) | 58% |
| **WX001** (50 mg/kg, PO, BID) | 102% |

Conclusion: **WX001** can inhibit tumor growth in a dose-dependent manner at three doses of 12.5 mg/kg, 25 mg/kg and 50 mg/kg. During the administration, the body weight of animals is not observed to decrease significantly, and the tolerance is good.

### Assay example 6. In vivo PK study in SD rats

### 1. Purpose of the assay:

Male SD rats were used as assay animals. After a single administration, the plasma concentration of the compound was measured and the pharmacokinetic behavior was evaluated.

### 2. Procedure of the assay:

Six healthy adult male SD rats were selected, wherein 3 rats were in an intravenous injection group and 3 rats were in an oral group. The vehicle in the intravenous injection group was 5% DMSO+95% (20% HP-β-CD). The compound to be assayed was mixed with an appropriate amount of vehicle for intravenous injection, vortexed and sonicated to prepare a clear solution of 0.2 mg/mL. The clear solution was filtered by a microporous membrane, and then ready for use. The vehicle in the oral group was 5% DMSO+95% (20% HP-β-CD). The compound to be assayed was mixed with the vehicle, vortexed and sonicated to prepare a solution of 1 mg/mL. SD rats were administered 1 mg/kg intravenously or 10 mg/kg orally, and then whole blood was collected for a certain period. Plasma was prepared. The drug concentration was analyzed by LC-MS/MS method, and the pharmacokinetic parameters were calculated by Phoenix WinNonlin software (Pharsight, USA).

Note: DMSO: dimethyl sulfoxide; HP-β-CD: hydroxypropyl-β-cyclodextrin.

3. The assay results are shown in Table 24:

**Table 24: Results of the PK assay of the compound**

| Compound | Cₘₐₓ (nM) | F% | Oral DNAUC (nM.h/mpk) | Vdₛₛ (L/kg) | Cl (mL/min/kg) | T_{1/2} (h) |
|---|---|---|---|---|---|---|
| **WX001** | 2500 | 49% | 1445.79 | 1.08 | 13.4 | 3.77 |

| | | | | | | |
|---|---|---|---|---|---|---|
| Note: Cₘₐₓ is maximum concentration; F% is oral bioavailability; DNAUC is AUC_{PO}/Dose, AUC_{PO} is oral exposure, and Dose is drug dose; Vdₛₛ is volume of distribution; Cl is clearance rate; and T_{1/2} is half-life. | | | | | | |

Conclusion: The compound of the present disclosure exhibits excellent oral exposure and bioavailability.

### Assay example 7. In vivo PK study in cynomolgus monkeys

### 1. Purpose of the assay:

Male cynomolgus monkeys were used as assay animals. After a single administration, the plasma concentration of the compound was measured and the pharmacokinetic behavior was evaluated.

### 2. Procedure of the assay:

Five healthy adult male cynomolgus monkeys were selected, wherein 2 animals were in an intravenous injection group, and 3 animals were in an oral group. The vehicle in the intravenous injection group was 5% DMSO+95% (20% HP-β-CD). The compound to be assayed was mixed with an appropriate amount of vehicle for intravenous injection, and dissolved with stirring to prepare a clear solution of 0.4 mg/mL. The clear solution was filtered by a microporous membrane, and then ready for use. The vehicle in the oral group was 5% DMSO+95% (20% HP-β-CD). The compound to be assayed was mixed with the vehicle, and dissolved with stirring to prepare a solution of 0.3 mg/mL. Cynomolgus monkeys were administered 1 mg/kg intravenously or 3 mg/kg orally, and then whole blood was collected for a certain period. Plasma was prepared. The drug concentration was analyzed by LC-MS/MS method, and the pharmacokinetic parameters were calculated by Phoenix WinNonlin software (Pharsight, USA).

Note: DMSO: dimethyl sulfoxide; HP-β-CD: hydroxypropyl-β-cyclodextrin.

3. The assay results are shown in Table 25:

**Table 25: Results of the PK assay of the compound**

| Compound | Cₘₐₓ (nM) | F% | Oral DNAUC (nM.h/mpk) | Vdₛₛ (L/kg) | Cl (mL/min/kg) | T_{1/2} (h) |
|---|---|---|---|---|---|---|
| **WX001** | 921 | 50% | 1152.35 | 1.98 | 16.1 | 2.41 |

| | | | | | | |
|---|---|---|---|---|---|---|
| Note: Cₘₐₓ is maximum concentration; F% is oral bioavailability; DNAUC is AUC_{PO}/Dose, AUC_{PO} is oral exposure, and Dose is drug dose; Vdₛₛ is volume of distribution; Cl is clearance rate; and T_{1/2} is half-life. | | | | | | |

Conclusion: The compound of the present disclosure exhibits excellent oral exposure and bioavailability.

### Assay example 8. In vivo PK study in beagle dogs

### 1. Purpose of the assay:

Male beagle dogs were used as assay animals. After a single administration, the plasma concentration of the compound was measured and the pharmacokinetic behavior was evaluated.

### 2. Procedure of the assay:

Five healthy adult male beagle dogs were selected, wherein 2 were in an intravenous injection group, and 3 were in an oral group. The vehicle in the intravenous injection group was 5% DMSO+95% (20% HP-β-CD). The compound to be assayed was mixed with an appropriate amount of vehicle for intravenous injection, and dissolved with stirring to prepare a clear solution of 0.4 mg/mL. The clear solution was filtered by a microporous membrane, and then ready for use. The vehicle in the oral group was 5% DMSO+95% (20% HP-β-CD). The compound to be assayed was mixed with the vehicle, and dissolved with stirring to prepare a solution of 0.3 mg/mL. Beagle dogs were administered 1 mg/kg intravenously or 3 mg/kg orally, and then whole blood was collected for a certain period. Plasma was prepared. The drug concentration was analyzed by LC-MS/MS method, and the pharmacokinetic parameters were calculated by Phoenix WinNonlin software (Pharsight, USA).

Note: DMSO: dimethyl sulfoxide; HP-β-CD: hydroxypropyl-β-cyclodextrin.

3. The assay results are shown in Table 26:

**Table 26: Results of the PK assay of the compound**

| Compound | Cₘₐₓ (nM) | F% | Oral DNAUC (nM.h/mpk) | Vdₛₛ (L/kg) | Cl (mL/min/kg) | T_{1/2} (h) |
|---|---|---|---|---|---|---|
| **WX001** | 1769 | 60% | 2307.32 | 1.29 | 10.1 | 3.48 |

| | | | | | | |
|---|---|---|---|---|---|---|
| Note: Cₘₐₓ is maximum concentration; F% is oral bioavailability; DNAUC is AUC_{PO}/Dose, AUC_{PO} is oral exposure, and Dose is drug dose; Vdₛₛ is volume of distribution; Cl is clearance rate; and T_{1/2} is half-life. | | | | | | |

Conclusion: The compound of the present disclosure exhibits excellent oral exposure and bioavailability.

### Assay example 9. hERG assay

### 1. Purpose of the assay:

A fully automated patch-clamp method was used to assay the effect of the compound on hERG potassium channel (human Ether-a-go-go Related Gene potassium channel) current.

### 2. Method of the assay:

**2.1 Cell preparation**

CHO-hERG cells were cultured in a 175 cm² culture flask. When cells grew to a density of 60~80%, the culture medium was removed. Cells were washed once with 7 mL of PBS (Phosphate Buffered Saline), and then 3 mL of Detachin was added for digestion. After the digestion was completed, 7 mL of culture medium was added for neutralization, and then the mixture was centrifuged. The supernatant was aspirated, and then 5 mL of culture medium was added to resuspend the cells and ensure that the cell density was 2~5×10⁶/mL.

### 2.2 Solution preparation

Extracellular solution formulation (mM): 140 NaCl, 5 KCl, 1 CaCl₂, 1.25 MgCl₂, 10 HEPES and 10 Glucose; the formulation was adjusted to pH of 7.4 with NaOH.

Intracellular solution formulation (mM): 140 KCl, 1 MgCl₂, 1 CaCl₂, 10 EGTA and 10 HEPES; the formulation was adjusted to pH of 7.2 with KOH.

### 2.3 Electrophysiological recording process

The single-cell high-impedance sealing and whole-cell pattern formation processes were all automatically performed by a Qpatch instrument. After obtaining the whole-cell recording mode, the cells were clamped at -80 mV. The cells were applied successively with a pre-voltage of -50 mV for 50 milliseconds and a depolarizing stimulus of +40 mV for 5 seconds, then repolarized to -50 mV for 5 seconds, and then back to -80 millivolts. This voltage stimulation was applied every 15 seconds, and recorded for 2 minutes. The extracellular solution was then given, and recorded for 5 minutes. Then a drug administration process started. The compound concentration started from the lowest assay concentration, and each assay concentration was given for 2.5 minutes. After all concentrations were given successively, a positive control compound 3 M Cisapride was given. At least 3 cells were assayed for each concentration (n ≥ 3).

### 2.4 Compound preparation

20.00 mM mother liquor of the compound was diluted with DMSO. 10 µL of the mother liquor of the compound was added to 20 µL of DMSO solution, and serially diluted 3-fold to 6 DMSO concentrations. 4 µL of the compound with 6 DMSO concentrations was respectively added to 396 µL of the extracellular solution, and serially diluted 100-fold to 6 intermediate concentrations. 80 µL of the compound with 6 intermediate concentrations was respectively added to 320 µL of the extracellular solution, and serially diluted 5-fold to the final concentration to be assayed. The highest assayed concentration was 40 µM, and there was a total of 6 concentrations: 40, 13.3, 4.4, 1.48, 0.494 and 0.165 µM, respectively. The DMSO content in the final assay concentration did not exceed 0.2%. This concentration of DMSO had no effect on the hERG potassium channel. All dilutions in the compound preparation were performed by a Bravo instrument.

### 2.5 Data analysis

Assay data were analyzed by GraphPad Prism 5.0 software.

### 2.6 Quality control

Environment: Humidity 20~50%, temperature 22~25°C
Reagent: The used assay reagents were purchased from Sigma, with a purity of >98%.
Assay data in the report must meet the following standards:
Whole cell sealing impedance > 100 MΩ
Tail current amplitude > 300 pA
Pharmacological parameters:

The inhibitory effect of Cisapride with multiple concentrations on the hERG channel was mesured as a positive control.

3. The assay result is shown in Table 27:

**Table 27: Assay result of the compound against hERG**

| Compound | **IC50** (µM) |
|---|---|
| **WX001** | > 40 |

Conclusion: The compound of the present disclosure has a weak inhibitory effect on hERG potassium channel current, thereby lowering the risk of cardiotoxicity and improving safety.

### Assay example 10. Assay of plasma protein binding (PPB)

### 1. Purpose of the assay:

The binding degree of the assay compound to human/mouse/rat/canine/monkey plasma albumin was studied.

### 2. Procedure of the assay:

1) Matrix preparation: on the day of the assay, plasma was thawed in cold water and centrifuged at 3220 rpm for 5 min to remove all clots. The pH of the resulting plasma was measured and adjusted to 7.4 ± 0.1 using 1% phosphoric acid or 1N sodium hydroxide as needed.
2) Dilution procedure for the assay compound: the assay compound was dissolved in dimethyl sulfoxide (DMSO) to prepare stock solutions with concentrations of 10 mM and 2 mM, respectively. A 40 µM working solution was prepared by diluting 2 µL of stock solution (2 mM) with 98 µL of DMSO. A 400 µM working solution of the control compound was prepared by diluting 10 µL of stock solution with 240 µL of DMSO. The working solution of the compound (5 µL) was mixed well with a blank matrix (995 µL) at a ratio of 1:200 to prepare a loading matrix.
3) Analysis steps:
   a) An equal volume of 30 µL of loading matrix (n=2) was transferred to a sample collection plate to prepare a time 0 (T0) sample for residue determination. The sample was immediately matched with the corresponding blank buffer to a final volume of 60 µL, and the volume ratio of plasma to buffer in each well was 1:1. Then, 60 µL of 4% H₃PO₄ in H₂O and 480 µL of stop solution containing the internal standard were added to the T0 sample of the assay compound. They were then stored with other samples at 2-8°C for further processing.
   b) The remaining plasma samples were pre-incubated in a carbon dioxide incubator at 37 ± 1°C for 30 min. Protein-free samples (F samples) and samples loaded with matrix (230 µL) were all transferred into polycarbonate tubes (n = 2) and ultracentrifuged at 37°C and 155,000 × g (35,000 rpm) for 4 h.
   c) To prepare T samples (assay samples), an additional matrix-containing sample was transferred to a separate 96-well plate (sample incubation plate) and incubated at 37°C for 4 h.
   d) At the end of centrifugation, 30 µL of protein-free samples and 30 µL of T samples were transferred from the second layer of the supernatant (below the top layer) to a new sample collection plate. Each sample was mixed with the corresponding blank buffer or matrix to a final volume of 60 µL with a matrix:buffer volume ratio of 1:1. 60 µL of 4% H₃PO₄ aqueous solution and 480 µL of stop solution (with internal standard) were added to all samples. The mixture was centrifuged at 4000 rpm for 20 min and 100 µL of supernatant from each sample was analyzed by LC-MS/MS.

### 3. The assay results are shown in Table 28:

**Table 28: Assay results of the plasma protein binding of the compound**

| Compound | Plasma protein binding rate (unbound%) | | | | |
|---|---|---|---|---|---|
| | Human | Mouse | Rat | Canine | Monkey |
| **WX001** | 15.9% | 11.7% | 8.4% | 14.3% | 14.2% |

Conclusion: The compound of the present disclosure has moderate plasma protein binding.

## Claims

1. A compound represented by formula (I) or a pharmaceutically acceptable salt thereof, 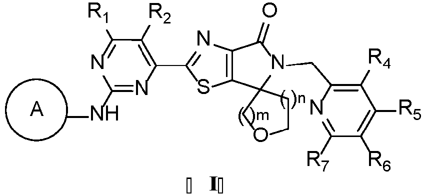 wherein
R₁ and R₂ are each independently selected from H and C₁₋₃ alkyl, wherein the C₁₋₃ alkyl is optionally substituted with 1, 2 or 3 Rₐ;
R₄, R₅, R₆, and R₇ are each independently selected from H, F, Cl, Br, I and C₁₋₃ alkyl, wherein the C₁₋₃ alkyl is optionally substituted with 1, 2 or 3 R_{c};
n is 0 or 1;
m is 1 or 2;
ring A is selected from pyrazolyl and tetrahydropyranyl, wherein the pyrazolyl and tetrahydropyranyl are optionally substituted with 1, 2 or 3 R_{d};
Rₐ and R_{c} are each independently selected from D, F, Cl, Br and I;
R_{d} is selected from F, Cl, Br, I, C₁₋₃ alkyl and C₁₋₃ alkoxy, wherein the C₁₋₃ alkyl and C₁₋₃ alkoxy are optionally substituted with 1, 2 or 3 R;
R is selected from F, Cl, Br and I.

2. The compound according to claim 1 or a pharmaceutically acceptable salt thereof, wherein R₁ and R₂ are each independently selected from H, CH₃ and CH₂CH₃, wherein the CH₃ and CH₂CH₃ are optionally substituted with 1, 2 or 3 Rₐ;
alternatively, wherein R₁ and R₂ are each independently selected from H, CH₃, CHF₂, CD₃ and CH₂CH₃.

3. The compound according to claim 1 or a pharmaceutically acceptable salt thereof, wherein
R₄, R₅, R₆, and R₇ are each independently selected from H, F, Cl, Br, I and CH₃, wherein the CH₃ is optionally substituted with 1, 2 or 3 R_{c};
alternatively, wherein R₄, R₅, R₆ and R₇ are each independently selected from H, F, Cl, Br, I and CH₃.

4. The compound according to claim 1 or a pharmaceutically acceptable salt thereof, wherein the compound has one or more of the following definitions:
i) R_{d} is selected from F, Cl, Br, I, CH₃ and OCH₃, wherein the CH₃ and OCH₃ are optionally substituted with 1, 2 or 3 R;
ii) R_{d} is selected from F, Cl, Br, I, and CH₃, wherein the CH₃ is optionally substituted with 1, 2 or 3 R;
iii) R_{d} is selected from CH₃ and OCH₃;
iv) R_{d} is selected from CH₃;
v) ring A is selected from pyrazolyl, wherein the pyrazolyl is optionally substituted with 1, 2 or 3 R_{d};
vi) ring A is selected from 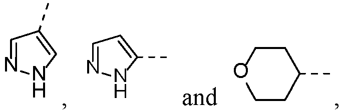 wherein the 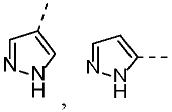 and 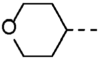 are optionally substituted with 1, 2 or 3 R_{d};
vii) ring A is selected from 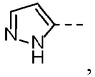 wherein the 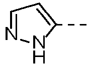 is optionally substituted with 1, 2 or 3 R_{d};
viii) ring A is selected from 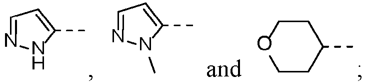
ix) ring A is selected from 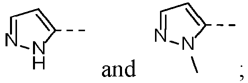
x) the structural moiety 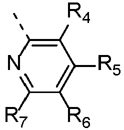 is selected from 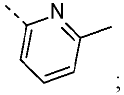
xi) R_{d} is selected from F, Cl, Br, I, and C₁₋₃ alkyl, wherein the C₁₋₃ alkyl is optionally substituted with 1, 2 or 3 R.

5. The compound according to any one of claims 1 to 3 or a pharmaceutically acceptable salt thereof, wherein the compound is selected from 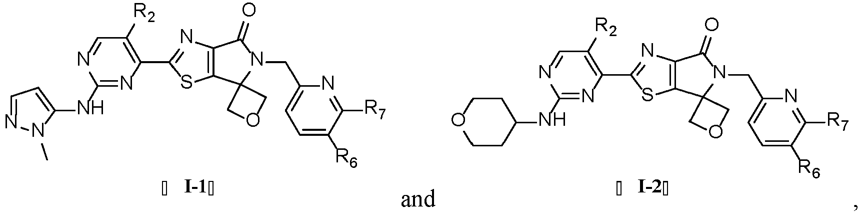 wherein
R₂ is as defined in claim 1 or 2;
R₆ and R₇ are as defined in claim 1 or 3.

6. The compound according to claim 1 or a pharmaceutically acceptable salt thereof, wherein the compound has the following structure:

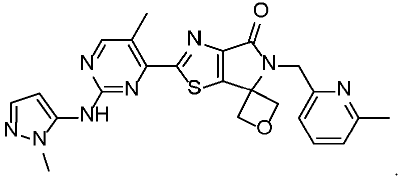

7. A crystal form A of the compound according to claim 6, which is **characterized by** an X-ray powder diffraction pattern having characteristic diffraction peaks at 2θ angles of: 10.2080±0.2000°, 18.8429±0.2000°, 20.6217±0.2000°;

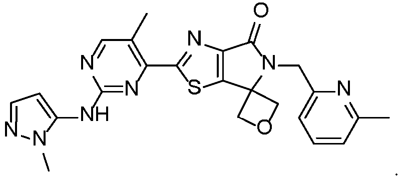

8. The crystal form A according to claim 7, wherein the X-ray powder diffraction pattern has characteristic diffraction peaks at 2θ angles of 10.2080±0.2000°, 18.8429±0.2000°, 20.6217±0.2000°, 25.0767±0.2000°, 25.4797±0.2000°.

9. The crystal form A according to claim 8, wherein the X-ray powder diffraction pattern has characteristic diffraction peaks at 2θ angles of 10.2080±0.2000°, 15.2687±0.2000°, 17.6747±0.2000°, 18.8429±0.2000°, 20.6217±0.2000°, 21.0531±0.2000°, 25.0767±0.2000°, 25.4797±0.2000°.

10. The crystal form A according to claim 9, wherein the X-ray powder diffraction pattern has characteristic diffraction peaks at 2θ angles of 10.2080±0.2000°, 14.4684±0.2000°, 15.2687±0.2000°, 17.6747±0.2000°, 18.8429±0.2000°, 20.6217±0.2000°, 21.0531±0.2000°, 21.5713±0.2000°, 22.0420±0.2000°, 22.4540±0.2000°, 25.0767±0.2000°, 25.4797±0.2000°.

11. The crystal form A according to claim 10, wherein the X-ray powder diffraction pattern has characteristic diffraction peaks at 2θ angles of 10.2080°, 10.4856°, 14.4684°, 15.0133°, 15.2687°, 15.9518°, 16.6214°, 17.6747°, 17.9514°, 18.4703°, 18.8429°, 19.1531°, 20.6217°, 21.0531°, 21.2894°, 21.5713°, 22.0420°, 22.4540°, 25.0767°, 25.4797°, 26.3255°, 26.9544°;
alternatively, which has an XRPD pattern substantially as shown in FIG. 1.

12. The crystal form A according to any one of claims 7 to 11, which has a differential scanning calorimetry curve having an onset of an endothermic peak at 241.0±3.0°C;
alternatively, which has a DSC curve as shown in FIG. 2.

13. The crystal form A according to any one of claims 7 to 12, which has a thermogravimetric analysis curve having a weight loss of up to 0.83% at 150.0±3.0°C;
alternatively, which has a TGA curve as shown in FIG. 3.

14. A compound according to any one of claims 1 to 6 or a pharmaceutically acceptable salt thereof or a crystal form A according to any one of claims 7 to 13, for use in the treatment of a solid tumor.

## Patentansprüche

1. Verbindung dargestellt durch Formel (I) oder ein pharmazeutisch verträgliches Salz davon, 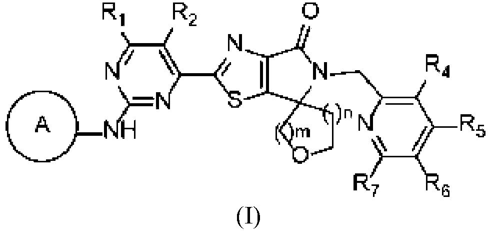 wobei
R₁ und R₂ jeweils unabhängig ausgewählt sind aus H und C₁₋₃- A lkyl, wobei C₁₋₃- Alkyl optional mit 1, 2 or 3 Rₐ substituiert ist;
R₄, R₅, R₆ und R₇ jeweils unabhängig ausgewählt sind aus H, F, Cl, Br, I und C₁₋₃-Alkyl, wobei C₁₋₃- Alkyl optional mit 1, 2 or 3 R_{c} substituiert ist;
n 0 oder 1 ist;
m 1 oder 2 ist;
Ring A ausgewählt ist aus Pyrazolyl und Tetrahydropyranyl, wobei Pyrazolyl und Tetrahydropyranyl optional mit 1, 2 or 3 R_{d} substituiert sind;
Rₐ und R_{c} jeweils unabhängig ausgewählt sind aus D, F, Cl, Br und I;
R_{d} ausgewählt ist aus F, Cl, Br, I, C₁₋₃-Alkyl und C₁₋₃-Alkoxy, wobei C₁₋₃-Alkyl und C₁₋₃-Alkoxy optional mit 1, 2 or 3 R substituiert sind;
R ausgewählt ist aus F, Cl, Br und I.

2. Die Verbindung gemäß Anspruch 1 oder ein pharmazeutisch verträgliches Salz davon, wobei R₁ und R₂ jeweils unabhängig ausgewählt sind aus H, CH₃ und CH₂CH₃, wobei CH₃ und CH₂CH₃ optional mit 1, 2 or 3 Rₐ substituiert sind;
alternativ, wobei R₁ und R₂ jeweils unabhängig ausgewählt sind aus H, CH₃, CHF₂, CD₃ und CH₂CH₃.

3. Die Verbindung gemäß Anspruch 1 oder ein pharmazeutisch verträgliches Salz davon, wobei R₄, R₅, R₆ und R₇ jeweils unabhängig ausgewählt sind aus H, F, Cl, Br, I und CH₃, wobei CH₃ optional mit 1, 2 or 3 R_{c} substituiert ist;
alternativ, wobei R₄, R₅, R₆ und R₇ jeweils unabhängig ausgewählt sind aus H, F, Cl, Br, I und CH₃.

4. Die Verbindung gemäß Anspruch 1 oder ein pharmazeutisch verträgliches Salz davon, wobei auf die Verbindung gemäß Anspruch 1 eine oder mehrere der folgenden Definitionen zutrifft:
i) R_{d} ausgewählt ist aus F, Cl, Br, I, CH₃ und OCH₃, wobei CH₃ und OCH₃ optional mit 1, 2 or 3 R substituiert sind;
ii) R_{d} ausgewählt ist aus F, Cl, Br, I und CH₃, wobei CH₃ optional mit 1, 2 or 3 R substituiert ist;
iii) R_{d} ausgewählt ist aus CH₃ und OCH₃;
iv) R_{d} ausgewählt ist aus CH₃;
v) Ring A ausgewählt ist aus Pyrazolyl, wobei Pyrazolyl optional mit 1, 2 or 3 R_{d} substituiert ist;
vi) Ring A ausgewählt ist aus 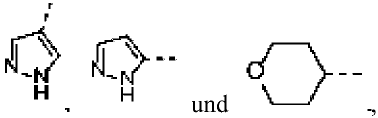 wobei 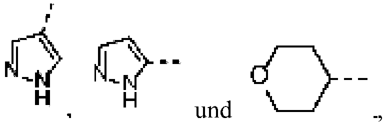 optional mit 1, 2 or 3 R_{d} substituiert sind;
vii) Ring A ausgewählt ist aus 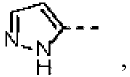 wobei 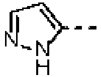 optional mit 1, 2 or 3 R_{d} substituiert ist;
viii) Ring A ausgewählt ist aus 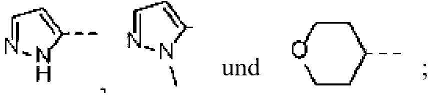
ix) Ring A ausgewählt ist aus 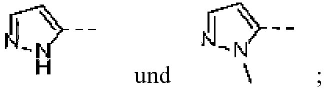
x) die strukturelle Einheit 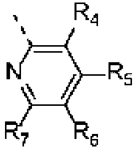 ausgewählt ist aus 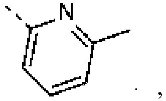
xi) R_{d} ausgewählt ist aus F, Cl, Br, I und C₁₋₃-Alkyl, wobei C₁₋₃-Alkyl optional mit 1, 2 or 3 R substituiert ist.

5. Die Verbindung gemäß einem der Ansprüche 1 bis 3 oder ein pharmazeutisch verträgliches Salz davon, wobei die Verbindung ausgewählt ist aus 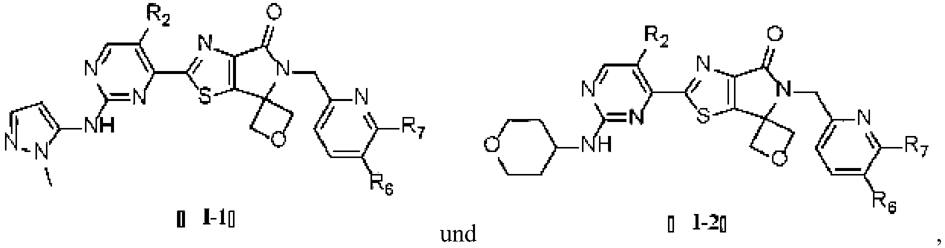 wobei
R₂ so ist, wie in Anspruch 1 oder 2 definiert;
R₆ und R₇ so sind, wie in Anspruch 1 oder 3 definiert.

6. Die Verbindung gemäß Anspruch 1 oder ein pharmazeutisch verträgliches Salz davon, wobei die Verbindung die folgende Struktur hat:

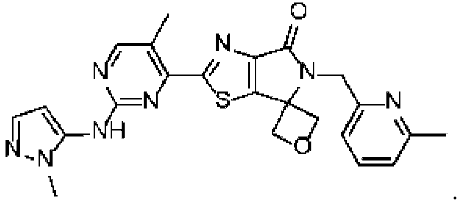

7. Kristalline Form A der Verbindung gemäß Anspruch 6, welche durch ein Röntgenpulverdiffraktionsmuster (X-ray powder diffraction pattern, XRPD) charakterisiert ist, das charakteristische Diffraktionspeaks bei 2θ Winkeln von: 10,2080±0,2000°, 18,8429±0,2000°, 20,6217±0,2000° aufweist;

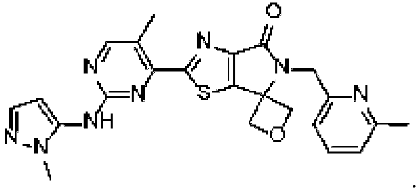

8. Die kristalline Form A gemäß Anspruch 7, wobei das Röntgenpulverdiffraktionsmuster (X-ray powder diffraction pattern, XRPD) charakteristische Diffraktionspeaks bei 2θ Winkeln von 10,2080±0,2000°, 18,8429±0,2000°, 20,6217±0,2000°, 25,0767±0,2000°, 25,4797±0,2000° aufweist.

9. Die kristalline Form A gemäß Anspruch 8, wobei das Röntgenpulverdiffraktionsmuster (X-ray powder diffraction pattern, XRPD) charakteristische Diffraktionspeaks bei 2θ Winkeln von 10,2080±0,2000°, 15,2687±0,2000°, 17,6747±0,2000°, 18,8429±0,2000°, 20,6217±0,2000°, 21,0531±0,2000°, 25,0767±0,2000°, 25,4797±0,2000° aufweist.

10. Die kristalline Form A gemäß Anspruch 9, wobei das Röntgenpulverdiffraktionsmuster (X-ray powder diffraction pattern, XRPD) charakteristische Diffraktionspeaks bei 2θ Winkeln von 10,2080±0,2000°, 14,4684±0,2000°, 15,2687±0,2000°, 17,6747±0,2000°, 18,8429±0,2000°, 20,6217±0,2000°, 21,0531±0,2000°, 21,5713±0,2000°, 22,0420±0,2000°, 22,4540±0,2000°, 25,0767±0,2000°, 25,4797±0,2000° aufweist.

11. Die kristalline Form A gemäß Anspruch 10, wobei das Röntgenpulverdiffraktionsmuster (X-ray powder diffraction pattern, XRPD) charakteristische Diffraktionspeaks bei 2θ Winkeln von 10,2080°, 10,4856°, 14,4684°, 15,0133°, 15,2687°, 15,9518°, 16,6214°, 17,6747°, 17,9514°, 18,4703°, 18,8429°, 19,1531°, 20,6217°, 21,0531°, 21,2894°, 21,5713°, 22,0420°, 22,4540°, 25,0767°, 25,4797°, 26,3255°, 26,9544° aufweist;
alternativ, welche ein Röntgenpulverdiffraktionsmuster (XRPD pattern), im Wesentlichen wie in FIG. 1 gezeigt, aufweist.

12. Die kristalline Form A gemäß einem der Ansprüche 7 bis 11, welche eine Differenzkalorimetrie-Kurve mit einem Beginn eines endothermen Peaks bei 241,0±3,0°C aufweist;
alternativ, welche eine DSC Kurve, wie in FIG. 2 gezeigt, aufweist.

13. Die kristalline Form A gemäß einem der Ansprüche 7 bis 12, welche eine thermogravimetrische Analysekurve mit einem Gewichtsverlust von bis zu 0,83% bei 150,0±3,0°C aufweist;
alternativ, welche eine TGA Kurve, wie in FIG. 3 gezeigt, aufweist.

14. Verbindung gemäß einem der Ansprüche 1 bis 6 oder ein pharmazeutisch verträgliches Salz davon, oder kristalline Form A gemäß einem der Ansprüche 7 bis 13 zur Verwendung in der Behandlung eines soliden Tumors.

## Revendications

1. Composé représenté par la formule (I) ou un sel pharmaceutiquement acceptable de celui-ci, 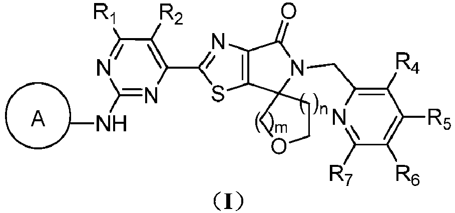 dans lequel
chacun de R₁ et R₂ est indépendamment choisi parmi H et alkyle en C₁ à C₃, lequel alkyle en C₁ à C₃ étant éventuellement substitué par 1, 2 ou 3 Rₐ ;
chacun de R₄, R₅, R₆ et R₇ est indépendamment choisi parmi H, F, Cl, Br, I et alkyle en C₁ à C₃, lequel alkyle en C₁ à C₃ étant éventuellement substitué par 1, 2 ou 3 R_{c} ;
n vaut 0 ou 1 ;
m vaut 1 ou 2 ;
le cycle A est choisi parmi pyrazolyle et tétrahydropyranyle, lesquels pyrazolyle et tétrahydropyranyle étant éventuellement substitués par 1, 2 ou 3 R_{d} ;
chacun de Rₐ et R_{c} est indépendamment choisi parmi D, F, Cl, Br et I ;
R_{d} est choisi parmi F, Cl, Br, I, alkyle en C₁ à C₃ et alcoxy en C₁ à C₃, lesquels alkyle en C₁ à C₃ et alcoxy en C₁ à C₃ étant éventuellement substitués par 1, 2 ou 3 R ;
R est choisi parmi F, Cl, Br et I.

2. Composé selon la revendication 1 ou un sel pharmaceutiquement acceptable de celui-ci,
dans lequel chacun de R₁ et R₂ est indépendamment choisi parmi H, CH₃ et CH₂CH₃, lesquels CH₃ et CH₂CH₃ étant éventuellement substitués par 1, 2 ou 3 Rₐ ;
en variante dans lequel chacun de R₁ et R₂ est indépendamment choisi parmi H, CH₃, CHF₂, CD₃ et CH₂CH₃.

3. Composé selon la revendication 1 ou un sel pharmaceutiquement acceptable de celui-ci,
dans lequel chacun de R₄, R₅, R₆ et R₇ est indépendamment choisi parmi H, F, Cl, Br, I et CH₃, lequel CH₃ étant éventuellement substitué par 1, 2 ou 3 R_{c} ;
en variante, dans lequel chacun de R₄, R₅, R₆ et R₇ est indépendamment choisi parmi H, F, Cl, Br, I et CH₃.

4. Composé selon la revendication 1 ou un sel pharmaceutiquement acceptable de celui-ci, lequel composé a une ou plusieurs des définitions suivantes :
i) R_{d} est choisi parmi F, Cl, Br, I, CH₃ et OCH₃, lesquels CH₃ et OCH₃ étant éventuellement substitués par 1, 2 ou 3 R ;
ii) R_{d} est choisi parmi F, Cl, Br, I et CH₃, lequel CH₃ étant éventuellement substitué par 1, 2 ou 3 R ;
iii) R_{d} est choisi parmi CH₃ et OCH₃ ;
iv) R_{d} est choisi parmi CH₃ ;
v) le cycle A est choisi parmi un pyrazolyle, lequel pyrazolyle étant éventuellement substitué par 1, 2 ou 3 R_{d} ;
vi) le cycle A est choisi parmi 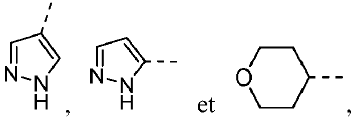 lesquels 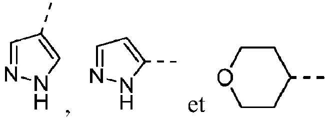 étant éventuellement substitués par 1, 2 ou 3 R_{d} ;
vii) le cycle A est choisi parmi 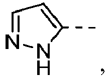 lequel 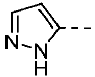 étant éventuellement substitué par 1, 2 ou 3 R_{d} ;
viii) le cycle A est choisi parmi 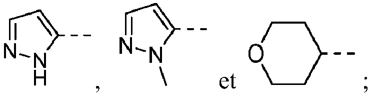
ix) le cycle A est choisi parmi 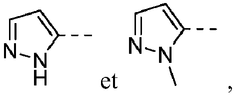
x) le fragment structurel 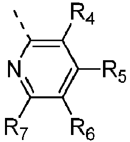 est choisi parmi 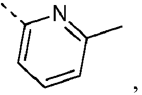
xi) R_{d} est choisi parmi F, Cl, Br, I et alkyle en C₁ à C₃, lequel alkyle en C₁ à C₃ étant éventuellement substitué par 1, 2 ou 3 R.

5. Composé selon l'une quelconque des revendications 1 à 3 ou un sel pharmaceutiquement acceptable de celui-ci, lequel composé est choisi parmi 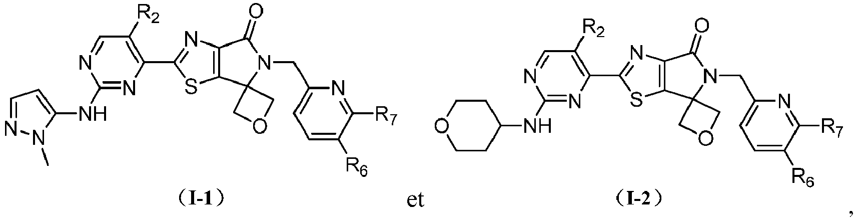 dans lesquels
R₂ est tel que défini dans la revendication 1 ou 2 ;
R₆ et R₇ sont tels que définis dans la revendication 1 ou 3.

6. Composé selon la revendication 1 ou un sel pharmaceutiquement acceptable de celui-ci, lequel composé ayant la structure suivante :

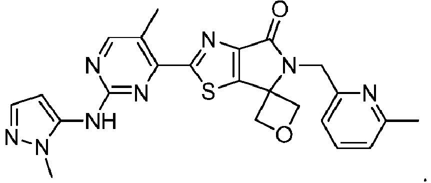

7. Forme cristalline A du composé selon la revendication 6, qui est **caractérisée par** un motif de diffraction des rayons X par la technique des poudres ayant des pics de diffraction caractéristiques à des angles 2θ de : 10,2080 ± 0,2000°, 18,8429 ± 0,2000°, 20,6217 ± 0,2000° ;

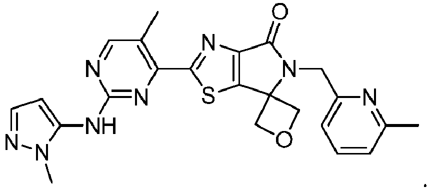

8. Forme cristalline A selon la revendication 7, dans laquelle le motif de diffraction des rayons X par la technique des poudres a des pics de diffraction caractéristiques à des angles 2θ de 10,2080 ± 0,2000°, 18,8429 ± 0,2000°, 20,6217 ± 0,2000°, 25,0767 ± 0,2000°, 25,4797 ± 0,2000°.

9. Forme cristalline A selon la revendication 8, dans laquelle le motif de diffraction des rayons X par la technique des poudres a des pics de diffraction caractéristiques à des angles 2θ de 10,2080 ± 0,2000°, 15,2687 ± 0,2000°, 17,6747 ± 0,2000°, 18,8429 ± 0,2000°, 20,6217 ± 0,2000°, 21,0531 ± 0,2000°, 25,0767 ± 0,2000°, 25,4797 ± 0,2000°.

10. Forme cristalline A selon la revendication 9, dans laquelle le motif de diffraction des rayons X par la technique des poudres a des pics de diffraction caractéristiques à des angles 2θ de 10,2080 ± 0,2000°, 14,4684 ± 0,2000°, 15,2687 ± 0,2000°, 17,6747 ± 0,2000°, 18,8429 ± 0,2000°, 20,6217 ± 0,2000°, 21,0531 ± 0,2000°, 21,5713 ± 0,2000°, 22,0420 ± 0,2000°, 22,4540 ± 0,2000°, 25,0767 ± 0,2000°, 25,4797 ± 0,2000°.

11. Forme cristalline A selon la revendication 10, dans laquelle le motif de diffraction des rayons X par la technique des poudres a des pics de diffraction caractéristiques à des angles 2θ de 10,2080°, 10,4856°, 14,4684°, 15,0133°, 15,2687°, 15,9518°, 16,6214°, 17,6747°, 17,9514°, 18,4703°, 18,8429°, 19,1531°, 20,6217°, 21,0531°, 21,2894°, 21,5713°, 22,0420°, 22,4540°, 25,0767°, 25,4797°, 26,3255°, 26,9544° ;
en variante, qui a un motif de XRPD sensiblement tel que représenté sur la Figure 1.

12. Forme cristalline A selon l'une quelconque des revendications 7 à 11, qui a une courbe d'analyse calorimétrique différentielle ayant un début de pic endothermique à 241,0 ± 3,0 °C ;
en variante qui a une courbe de DSC telle que représentée sur la Figure 2.

13. Forme cristalline A selon l'une quelconque des revendications 7 à 12, qui a une courbe d'analyse thermogravimétrique ayant une perte de poids allant jusqu'à 0,83 % à 150,0 ± 3,0 °C ;
en variante qui a une courbe de TGA telle que représentée sur la Figure 3.

14. Composé selon l'une quelconque des revendications 1 à 6 ou un sel pharmaceutiquement acceptable de celui-ci, ou forme cristalline A selon l'une quelconque des revendications 7 à 13, pour son utilisation dans le traitement d'une tumeur solide.
